# EUROPEAN PATENT SPECIFICATION

(11) **EP 2 072 029 B1**
(45) Date of publication and mention of the grant of the patent: **24.10.2012**
(21) Application number: 07123485.0
(22) Date of filing: 18.12.2007
(51) Int. Cl.: A61K 6/083, A61K 6/093, A61K 6/10

(54) **Dental Composition containing a Surfactant and an F-containing compound, Process of Production and Use thereof**
Dentalzusammensetzung mit einem Tensid und eine F-haltige Verbindung, Herstellungs- und Verwendungsverfahren dafür
Composition dentaire contenant un tensio-actif et un composant contenant du F, processus de production et utilisation associée

(43) Date of publication of application: 24.06.2009
(73) Proprietor: 3M Innovative Properties Company, St. Paul, MN 55133-3427 (US)
(72) Inventor: Bissinger, Peter, 86911, Diessen (DE); Maurer, Andreas, 86863, Langenneufnach (DE); Hoffmann, Henning, 86949, Windach (DE); Osswald, Peter, 86842, Türkenfeld (DE); Zech, Joachim, 86916, Kaufering (DE)
(74) Representative: Brem, Roland

(56) References cited:
- WO-A-2007/080071
- GB-A- 2 337 524
- US-A- 4 657 959

## Description

### Field of the Invention

The present invention relates to a curable dental composition comprising a surfactant and an F-containing compound. The dental composition can be used e.g. as impression material and/or for the production of crown and bridges.

### Background Art

Dental impression materials are well known in the art and have been applied for a long time. Such materials typically possess a variety of properties including a quick setting behavior, good dimensional stability and sufficient storage stability. Generally, the materials are provided in two components to be mixed prior to use and cure by a crosslinking-reaction.

One widely used class of impression materials is based on addition- or condensation crosslinking-reactions of polyorganosiloxane containing components, often referred to as VPS materials.

Dental impression materials containing polyorganosiloxane components are typically hydrophobic in nature. In order to make these materials more hydrophilic, the incorporation of surfactants has been proposed. Measuring the contact angle of a water drop on the surface of the mixed composition is an appropriate method to find out to which extend the composition has a hydrophilic or hydrophobic behavior.

US 5,064,891 describes an addition curable silicon composition comprising a silicone surfactant. The water contact angle measurement has been conducted on the cured material. The water contact angles measured 3 min (minutes) after the water drop has been applied on the surface of the composition was 60° and 65°, respectively.

EP 0 729 341 A1 (corresponding to US 5,750,589) discloses the use of a polyethercarbosilane for the hydrophilization of the impression material. The water contact angle measurement has been performed 30 min after curing of the material and values of 42° have been measured.

US 4,657,959 contains examples of compositions containing amphotheric and ionic surfactants. With regard to non-ionic fluor-containing surfactants, perfluorinated groups are attached to a polyether moiety via a polyvalent hydrocarbonyl linking group (e.g. - C₂H₄- or -SO₂NR- group).

Addition-curing silicone impression materials formulated with a combination of a non-ionic surfactant and a methylphenylpolysiloxane are known from US 5,907,002. The non-ionic surfactant according to this document can possess apart from a hydrophilic group a hydrophobic group which can be either an alkyl or a fluorocarbon group. Water contact angle between 28 and 60 degree have been achieved by these formulations in the cured state.

EP 1 290 998 A1 (corresponding to US 6,861,457 B2) describes addition-cured silicone impression material compositions, which contain an organopolysiloxane with at least two aliphatic hydrocarbon chains, a polyether with a minimum of one alkyl chain, an inorganic filling material, as well as a non-ionic nonfluorinated surfactant and/or a polyether modified silicon oil. Water contact angle measurements in the cured state were in the range of 56 to 65 degree, 1 second after setting of the drop.

EP 0 613 926 A1 (corresponding to US 5,569,691) discloses condensation-cured polyether impression materials, which contain at least one hydrophilic agent of the group of hydrophilic silicone oils, polyethylene glycol substituted fluorinated hydrocarbons, block copolymers of ethylenoxide and propylenoxide, fatty alcohol derivatives, alkylphenyl derivatives, fatty amines, amino oxide, fatty acid glycol or glycerine derivatives, fatty acids and fatty acid monoesters. The water contact angle measurement was performed 30 minutes after setting of the impression material and water contact angles in the range of 18 to 65 degree were found.

The use of mixtures of non-ionic surfactants to improve the wettability of the cured silicone impression material is described in US 2004/0236003 A1. Herein the non-ionic surfactants applied in the dental impression material are ethoxylated linear or branched hydrocarbon alcohols and/or acids.

In the above-mentioned patent documents the water contact angles have been exclusively determined on the cured rubber materials and, thus, the introduction of surfactants aimed mainly to improve the hydrophilicity of the set materials.

WO 2007/080071 A2 describes addition-cured dental impression materials based on silicones which provided hydrophilicity in the non-cured pasty state. By application of mixtures of fluorinated surfactants and silicone surfactants water contact angles of the pasty material below 10° were obtained 40 s (seconds) after mixing of the base and catalyst paste and 3 s after setting of the drop on the surface. The non-ionic fluorinated surfactants described contain at least one partly or per-fluorinated hydrocarbon rest, which is connected via an oxygen atom, an amino or a keto group, carboxylic ester group, a phosphoric acid ester and/or amide with an (poly)alkylenoxide radical, an carbohydrate radical, an aliphatic polyhydroxy radical or a nitrogen-containing heterocyclic compound or is at least a per- or partly fluorinated rest which comprise at least one amino-oxide rest.

However, not all of the F-containing surfactants suggested in WO 2007/080071 A2 were found to be suitable. E.g., it is not clear whether all them fulfil the requirements with respect to low toxicology and/or biocompatibility. Moreover, some of them where found to have a detrimental effect on other properties such as storage stability of the composition.

The present invention aims to provide an alternative or improved curable dental composition.

It can also be beneficial if the curable composition shows an improved flowing behaviour.

### Summary of the Invention

In one aspect, the present invention relates to a dental composition according to the claims comprising
a. a curable silicone polymer as component (A), the silicone polymer containing at least two functional groups capable of reacting with a SiH group in the presence of a hydrosilation catalyst,
b. a crosslinker compound containing at least two SiH groups as component (B),
c. a catalyst capable of catalyzing a hydrosilation reaction as component (C),
d. a surfactant as component (D),
e. at least one F-containing compound as component (E), wherein the F-containing compound has the following formula
   T₁-X-[(O-CF₂-CF₂)ᵤ-(O-CF₂)ᵥ-(O-CF(CF₃)-CF₂)_{w}-(O-CF₂-CF₂-CF₂)ₓ-O]-X-T₂
   with u = 0 to 8, v = 0 to 8, w = 0 to 8 and x =0 to 8 and u+v+w+x ≥ 1,
wherein T₁ and T₂ can be equal or different and are independently selected from - COOR, -CH₂OH, -CF₂OR, -CHFOH, -CHFOR, -CH₂OR or -F with R being a linear or branched alkyl rest (C1 to C9), aryl rest (C1 to C9) or alkylaryl rest (C1 to C9), and
wherein X is selected from -(CF₂)₁₋₆-, -CF(CF₃)- and -CHF-CF₂-.

According to another aspect, the invention features a kit of parts comprising a base paste and a catalyst paste separated from each other before use, wherein the base paste comprises components (A) and (B) and the catalyst paste comprises component (C) or (C) and (A) and wherein component (D) and/or (E) and the other optional components can be present either in the base paste or the catalyst paste or the base paste and the catalyst paste.

A further aspect of the invention is directed to a method or producing a dental composition comprising the step of combining the F-containing compound as described in the text of the invention with a hardenable matrix comprising a surfactant, preferably a Si-containing surfactant.

In yet another embodiment, the invention relates to a method of using the dental composition as described in the text of the invention as impression materials or for the preparation of crown and bridges.

Moreover, the invention is also directed to a method of using the F-containing component as described in the text of the invention for enhancing the hydrophilicity of a hardenable composition comprising a surfactant.

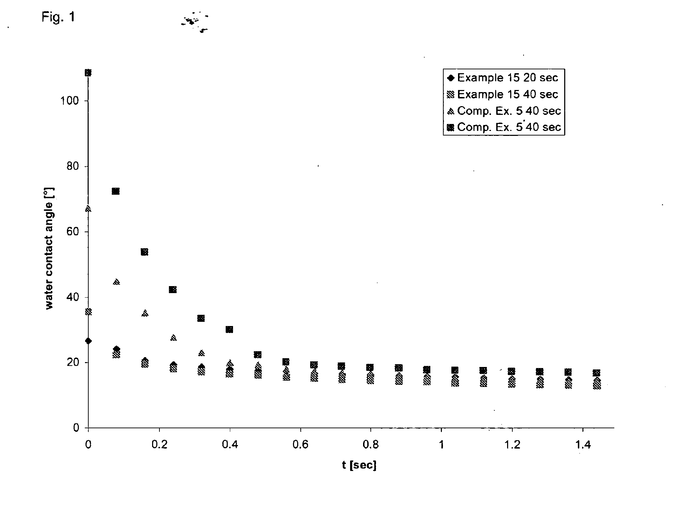

Fig.1 shows the time dependency of the water contact angle of exemplified compositions.

It has been found that the addition of an F-containing compound, which can be used in the present invention, to a common formulation of a silicon dental impression material has an impact on the wetting behavior (hydrophilicity) of the silicone containing dental composition. Typically, the wetting behavior of the dental composition with respect to hydrophilic surfaces (including human skin, mucosa, ginigiva and dental tooth structure) is improved.

This is surprising, because the F-containing compound, which can be used in the dental compositions of the present invention, does not possess a typical surfactant-like structure. That is, the F-containing compound does not have a "head-tail-structure", typical to most surfactants. A definition of the term "surfactant" and the respective structure of surfactants are given below.

The effect observed due to the addition of an F-containing compound to a composition containing e.g. a Si-containing surfactant is even more surprising in view of the fact that fluorinated polyethers have been known for many years and have been used as intermediates in the preparation of compositions to be applied to fibrous substrates to impart oil and/or water repellant properties. That is, these F-containing compounds have been used to make other materials hydrophobic.

It has been found, that a dental composition comprising an F-containing compound described in the present invention, but no surfactant does not show an improved wetting behavior in contrast to compositions containing a typical fluorinated surfactant, such as the ethoxylated nonionic fluorosurfactant Zonyl FSO-100 (DuPont).

In contrast to this, a dental composition containing a certain F-containing compound as described in the text of the invention in combination with a surfactant shows an improved wetting behavior, especially in the uncured state.

Thus, what has been observed is a synergistic effect between the F-containing compound described in the text of the invention and a surfactant.

Without wishing to be bound to a certain theory, a possible explanation for this surprising effect can be as follows: The F-containing compound might interact with the surfactant, e.g. by changing the micelle structure built by the molecules of the surfactant and thus causing an enlargement of the active surface of molecules of the surfactant. However, there might be other explanations as well.

Furthermore, with respect to some embodiments, it has also been revealed that a cured dental composition containing the F-containing compound as described in the text of the invention in combination with a surfactant shows an improved storage stability, e.g. compared with a dental composition containing the F-containing compound suggested in the examples of WO 2007/080071 A2.

Moreover, with respect to some embodiments, it has also been revealed that catalyst pastes comprising F-containing compounds according to the present invention show improved storage stability. Certain embodiments are more stable than compositions containing the F-containing compound suggested in the examples of WO 2007/080071 A2.

In addition, however, without wishing to be bound to this assumption, it is assumed that the F-containing compound(s) used in the dental materials of the present invention are environmentally friendly, that is, the composition is substantially free of fluorochemical components that may accumulate in the body of living organisms (e.g. less bio-accumulative and/or less toxic). The same may hold true for the respective metabolite(s) or the fluorochemical degradation products of the F-containing compounds used, as exemplified in US 2004/0124396 A1.

### Definitions

Within the description of the invention, the following terms are defined as follows: The term "compound" is a chemical substance which has a particular molecular identity or is made of a mixture of such substances, e.g., polymeric substances.

The term "hydrosilation" means the addition of an organosilicone hydride compound to a compound containing an aliphatic multiple bond (e.g., an olefinic or acetylenic unsaturation), preferably a vinyl group, -CH=CH₂.

By "paste" is meant a soft, viscous mass of solids dispersed in a liquid.

The term "silicone," as used herein, refers to a polymer having, for the most part, alternating silicon and oxygen atoms (i.e., a polysiloxane chemical structure) and having sufficient pendant functional groups to undergo a setting reaction in the presence of a crosslinker compound and a catalyst compound.

A "hardenable matrix" may be described as the components of a composition contributing to the formation of a network due to chemical interaction (e.g. formation of chemical bondings) between the components thereby leading to a significant change in rheological properties like viscosity.

The terms "vulcanizing, hardening, crosslinking, setting," are used interchangeable and refer to silicones that have as a common attribute the development of a crosslinked elastomer from relatively low molecular weight linear or branched polymers by means of a chemical reaction that simultaneously forms these crosslinks and effectively extends chain length at room temperature. "Room temperature vulcanizing" implies that the curing reaction can proceed at temperatures at or near 25°C. For example, the oral cavity of the mouth has an average temperature of approximately 32°C and is therefore near room temperature. Certain "high" temperature cured materials are designed to cure only at relatively high temperatures (e.g., >50°C or >100°C) and are stable (i.e., the curing reaction is retarded) at room temperature for prolonged periods.

The term "crosslinked polymer," as used herein, refers to polymers that react with the functional group or groups of the polymer chains to lengthen them and connect them, e.g., to form a crosslinked network characteristic of a silicone elastomer. In contrast to a thermoplastic polymer (i.e., a polymer that softens and flows upon heating) a crosslinked polymer, after crosslinking, is characteristically incapable of further flow.

The term "working time" as used herein, refers to the time between the initiation of the setting reaction (e.g., when the vinyl-containing organopolysiloxane, the organohydropolysiloxane, and the platinum catalyst are mixed) and the time the setting reaction has proceeded to the point at which it is no longer practical to perform further physical work upon the system, e.g., reform it, for its intended purpose. When the reaction has proceeded to this later point the material is said to have reached its "gel point." The working time preferably provides enough time to mix and place the composition into its desired form. For many dental impression compositions and applications the working time under conditions of use can be greater than about 30 s (seconds), or greater than about 1 min (minute), or greater than about 2 min. Thus, the working time is typically within a range of 30 s to 3 min or 1 min to 2 min. So-called "fast-setting" compositions typically have a shorter working time, e.g. less than about 2 min or less than about 1.5 min.

The terms "set time" or "setting time" as used herein, refer to the time at which sufficient curing has occurred so that essentially the material's final cured-state properties are obtained. For a silicone impression material the set time is that time at which one may remove the material from the surface being replicated without causing permanent deformation of the silicone material. The setting time may be approximated, for example, by measuring the torque of the reacting composition on an oscillatory rheometer. When the torque value reaches a maximum value the material is said to be fully set. An arbitrary torque value which is less than the typical maximum value (e.g. 90% of the maximum value) may alternatively be used as a practical approximation of the set time. In general, shorter setting times are preferred over longer setting times. For dental impression compositions the setting time occurs at a time preferably less than about 10 minutes after initiation of the reaction. More preferably the setting time is less than the sum of about 5 minutes plus the working time. Most preferably the setting time is just longer than the desired working time.

More specifically, the setting time is the time between positioning of the spoon with the dental material in the mouth of the patient and removal of the cured material, and can also be called the mouth residence time or period. Setting times of < about 3 min mouth residence time, preferably < about 2.5 min, and particularly preferably < about 2 min are desirable properties for the dentist working with situation impression materials. For example, the one-phase impression material Imprint^{™} (3M ESPE) has a setting time of about 5 minutes, while a typical alginate impression material such as Palgat^{™} (3M ESPE) has a setting time of about 4 min.

A "dental impression" may be described as an accurate representation of part or all of a person's dentition. It forms a "negative" of a person's hard dental tissue which can then be used to make a model (physical) of the dentition. This may be used for the fabrication of dentures, crowns or other prostheses. An impression is carried out by placing a liquid material into the mouth in a customised tray. The material then sets to become an elastic solid, and when removed from the mouth retains the shape of the teeth. Common materials used for dental impressions are sodium alginate, agar, polyethers including aziridino substituted polyether materials and silicones, both condensation-cured silicones and addition-cured silicones including polyvinyl siloxanes.

The term "dental tissue" includes the hard tooth substance (enamel and dentin), the gingival region (soft dental tissue) surrounding the hard tooth substance and hard tooth substance bearing orthodontic appliances.

Surfactants, also sometimes referred to as tensides, are wetting agents that are able to lower the surface tension of a liquid, allowing easier spreading, and lower the interfacial tension between two liquids.

Surfactants are usually organic compounds that are amphiphilic, meaning they contain both hydrophobic groups ("tails") and hydrophilic groups ("heads"). Typical examples include polyethyleneglycol-substituted fatty acids.

Usually, a surfactant can be classified by the presence of formally charged groups in its head. A nonionic surfactant has no charge groups in its head. The head of an ionic surfactant carries a net charge. If the charge is negative, the surfactant is more specifically called anionic; if the charge is positive, it is called cationic. If a surfactant contains a head with two oppositely charged groups, it is termed zwitterionic.

Surfactants typically reduce the surface tension of water by adsorbing at the liquid-gas interface. They also may reduce the interfacial tension between oil and water by adsorbing at the liquid-liquid interface. Many surfactants can also assemble in the bulk solution into aggregates. Some of these aggregates are known as micelles. The concentration at which surfactants begin to form micelles is known as the critical micelle concentration (CMC).

Surfactants can also be characterized by a "Hydrophobic Lipophilic Balance" value (HLB-value). Generally, with an increasing HLB-value a substance becomes more hydrophobic and in reverse more lipophilic. The measurement of the HLB-value of a certain substance can be accomplished by determining its aqueous solubility and cloud point, using e.g. the method described by H. Schott, J. Pharm. Science, 58, 1442, (1969). E.g. according to the product description, Silwett L-77 (a Si-containing surfactant) is said to have an estimated HLB value in the range of 5 to 8.

The "initial water contact angle" is defined as the contact angle of a water drop at the time 0 seconds (s) of the experiment (₀ₛ in degrees). The starting time of the experiment is defined as the time when the cannula, which is used for setting the water drop on a surface, does not have an influence on the shape of the water drop, i.e. when the cannula was removed from the water drop as soon as possible after placing of the water drop. Thus, ideally this time corresponds to the first contact of the water drop to the surface. Furthermore, the initial contact angle can be determined for any time after mixing of base paste and catalyst paste. The term "initial" does not refer to the time after mixing. The initial contact angle can be determined from Water Contact Angle Measurement as described in more detail in the Example section below, using e.g. a goniometer DSA 10 (Krüss). A low water contact angle typically indicates a better wettability.

The term "automixer-suitable impression material" relates to a multi-component impression material which can be dispensed, for example, from a two-component disposable cartridge through a static mixer, e.g., of SulzerMixpac Company (cf. US 5,464,131, EP 0 730 913 A1) or from film bags in dual-chamber reusable cartridges through a dynamic mixer, e.g., in the "Pentamix^{™}" and "Pentamix^{™} 2" devices of 3M ESPE Company (cf. US 5,286,105 and US 5,249,862).

A "dental compositions and dental articles" within the meaning of the present invention is a composition which is to be used in the dental field (including restorative and prosthodontic work) including the orthodontic area. In this respect, a dental composition typically does not contain hazardous substances. Commercially available products have to fulfil certain requirements such as those given in ISO 4823. Typically, those compositions cure or set at ambient conditions.

By a temporary or long term crown and bridge material is meant a material, which is used for the preparation of dental crowns and bridges containing hardenable monomers, including (meth)acrylates. These materials are typically used during the time period needed for making a permanent restoration. A typical time period ranges from a few days (e.g. 3 to 5) over weeks (1 to 3) to a few months (1 to 6). A long term crown and bridge material is typically used over a time period of 6 to 24 month. "Ambient conditions" mean the conditions which the inventive solution is usually subjected to during storage and handling. Ambient conditions may, for example, be a pressure of 900 to 1100 mbar, a temperature of -10 to 60 °C and a relative humidity of 10 to 100 %. In the laboratory ambient conditions are adjusted to 23 °C and 1013 mbar.

As used herein, "a", "an", "the", "at least one" and "one or more" are used interchangeably. The terms "comprises" or "contains" and variations thereof do not have a limiting meaning where these terms appear in the description and claims. Also herein, the recitations of numerical ranges by endpoints include all numbers subsumed within that range (e.g., 1 to 5 includes 1, 1.5, 2, 2.75, 3, 3.80, 4, 5, etc.).

Unless otherwise indicated, all numbers expressing quantities of ingredients, measurement of properties such as contrast ratio and so forth used in the specification and claims are to be understood as being modified in all instances by the term "about." Accordingly, unless indicated to the contrary, the numerical parameters set forth in the foregoing specification and attached claims are approximations that can vary depending upon the desired properties sought to be obtained by those skilled in the art utilizing the teachings of the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviations found in their respective testing measurements.

### Detailed Description of the Invention

Certain embodiments of the inventive curable dental composition can be characterized by at least one of the following features:
- Consistency (according to ISO 4823): 0, 1, 2 or 3 and/or
- Setting time: within about 15 min after mixing at ambient conditions (e.g. 23°C).

That is, the curable dental composition can show a comparable low viscous behaviour (consistency 3), a medium viscosity (consistency 1 or 2) or show a putty-like behaviour (consistency 0).

Certain embodiments of the cured inventive dental composition can be characterized by at least one of the following features:
- Tensile strength (according to DIN 53504): at least about 0,2 MPa, or at least 3,0 or at least 4,0,
- Elongation at break (according to DIN 53504): at least about 30 %, or at least 150 %, or at least 200 %,
- Recovery from deformation (according to ISO 4823): at least about 90 %, or at least 95 %, or at least 98 %,
- Shore A hardness (according to ISO 4823; 24h): at least 20 or at least 40.

The inventive dental composition can also be characterized by its water contact angle. Certain embodiments of the inventive composition have a water contact angle of less than about 20° or less than about 13° at a water drop age of 10 s, 60 s after mixing of the components (e.g. determined according to the method described in the Example section below).
Certain embodiments of the inventive composition have alternatively or in addition to the above water contact angle an initial water contact angle of less than about 80°, 40 s after mixing of the components (e.g. determined according to the method described in the Example section below).

The inventive composition contains as component (A) a curable silicone polymer containing at least two functional groups capable of reacting with a SiH group in the presence of a hydrosilation catalyst,

Typically, the curable silicone polymer is an organopolysiloxane with at least two pendant or terminal triorganosiloxy groups in which at least one of the three organic groups is a group with an ethylenically unsaturated double bond.

Generally, the groups with an ethylenically unsaturated double bond can be located on any monomeric unit of the organopolysiloxane. It is, however, preferred, that the groups with an ethylenically unsaturated double bond are located on or at least near the terminal, monomeric units of the polymer chain of the organopolysiloxane. In another embodiment, at least two of the groups with an ethylenically unsaturated double bond are located on the terminal monomeric units of the polymer chain.

The term "monomeric units" as used throughout the present text relates to repeating structural elements in the polymer that form the polymer backbone, unless expressly stated otherwise.

Preferred organopolysiloxanes of this general structure are represented by the following formula 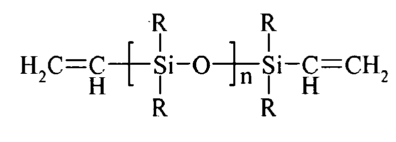 in which the radicals R, independently from each other, represent a non-substituted or substituted, monovalent hydrocarbon group with 1 to 6 C atoms, which is preferably free from aliphatic multiple bonds and where n generally can be chosen such that the viscosity of the organopolysiloxanes lies between 4 and 1,000,000 mPas or between 6 and 500,000 or between 10 and 100,000 mPas. The parameter n can, e.g., be in the range of 10 to 10,000.

Generally, the radicals R in the above formula can represent any non-substituted or substituted, monovalent hydrocarbon group with 1 to 6 C atoms. Non-substituted or substituted, monovalent hydrocarbon groups with 1 to 6 C atoms can be linear or, if the number of carbon atoms exceeds 2, branched or cyclic. Generally, the radicals R can be equipped with any type of substituent or substituents provided they do not interfere with any other constituents or substituents of the composition and do not interfere with the curing reaction.

The term "interfere" as used in the context of the present text relates to any influence of such a substituent on at least one of the other substituents or constituents of the composition or the curing reaction, or both, which might be detrimental to the properties of the hardened product.

The term "detrimental" as used in the context of the present text relates to a change of properties of the precursors or the cured product that negatively affect the usefulness of the precursors or the cured product in their intended use.

In another embodiment of the invention, at least about 50% of the radicals R are methyl groups. Examples of other radicals R that can be present in the organopolysiloxanes according to the above formula are ethyl, propyl, iso-propyl, n-butyl, iso-butyl, tert-butyl, the pentyl isomers, the hexyl isomers, vinyl, propenyl, iso-propenyl, 2- and 3-n-butenyl, the pentenyl isomers, the hexenyl isomers, fluorine substituted aliphatic radicals like 3,3,3-trifluoropropyl groups, cyclopentyl or cyclohexyl groups, cyclopentenyl or cyclohexenyl groups or aromatic or heteroaromatic groups like phenyl or substituted phenyl groups. Examples for such molecules are described in US 4,035,453,

The preparation of molecules according to the above-mentioned formula would generally be understood by the skilled person based upon the teachings of the prior art regarding similar molecules.

Particularly preferred are linear polydimethylsiloxanes according to the above formula having viscosities within the specified viscosity ranges and end groups comprising dimethylvinylsiloxy units and methyl groups as the radicals R.

A component (A) which can be employed according to the invention can consist of one type (A1) of organopolysiloxane. The organopolysiloxane can have a viscosity starting in the range of 5 to 1,000,000 mPas, or 10 to 500,000 mPas or 20 to 50,000 or 30 to 40,000 mPas.

It is, however, also possible that component (A) comprises two or more constituents, (A1), (A2) and so on, which can differ, e.g., in the chemical composition of their backbone, or their molecular weight, or their substituents or their viscosity, or any other differentiating feature or two or more of the above mentioned features.

In one embodiment of the invention the difference in viscosities of different constituents of component (A) can be higher than a factor of 2, e.g., higher than a factor of about 5, higher than a factor of 10, higher than a factor of 20, higher than a factor of 30, higher than a factor of 40, higher than a factor of 50, higher than a factor of 60, higher than a factor of 70, higher than a factor of 80, higher than a factor of 90 or higher than a factor of 100. The difference in viscosities can be even higher, e.g., higher than a factor of 200, higher than a factor of 300, higher than a factor of 500, higher than a factor of 800, higher than a factor of 1,000 or higher than a factor of 5,000, it should, however, preferably not exceed a value higher than a factor of 10,000. It should be kept in mind that the values mentioned above relate to a factor for the difference in viscosities, not the viscosity values themselve.

The viscosity can be measured using a Haake Rotovisco RV20 device (spindle MV, measuring cup NV). The viscosity is typically measured at 23 °C. After activation and rectification of the system, spindle MV is installed. Then the material to be measured is filled into the measuring cup NV. Without undue delay, the spindle is lowered into the measuring cup NV. The spindle should be covered by a layer of the material of a maximum thickness of 1 mm. The material to be measured is tempered for 20 min at 23 °C. The measurement is started by starting the spindle to turn and the viscosity values (mPas) are recorded starting 20 s after the start of measurement. Care must be exercised to ensure that the measuring cup NV does not rotate or move at any time. A value for the viscosity is obtained in mPas. The above mentioned method of measurement corresponds to DIN 53018-1.

If component (A) contains constituents of different viscosities, the ratio of the amount of constituent with the lowest viscosity to the amount of constituent with the highest viscosity can be chosen relatively freely, depending on the desired properties of the precursors and the cured resin. It can, however, be advantageous when the ratio of the amount of constituent with the lowest viscosity to the amount of constituent with the highest viscosity is within a range of from 1:20 to 20:1, especially 1:10 to 10:1 or 1:5 to 5:1. Good results can e.g. be obtained with ratios of from 1:3 to 3:1 or 1:2 to 2:1. It can furthermore be adequate in some cases, when the amount of constituent with the highest viscosity is about equal to or higher than the amount of constituent with the lowest viscosity, resulting in a value of from 0.9:1 to 3:1 for the ratio of the amount of constituent with the highest viscosity to the amount of constituent with the lowest viscosity. All of the ratios are based on the weight of the constituents.

In certain embodiment of the invention, the dental composition can comprise as part of component (A) multifunctional (including tri- or quadri-functional) ethylenically unsaturated compounds. These compounds might contribute to improve the tensile strength of the cured composition.

These compounds include the silane compounds described e.g. in WO 2004/098542 (component (H)) and the QM resins (quadric-functional unsaturated siloxane) described e.g. in US 2002/0193502, especially those mentioned in sections [0024] to [0026].

The component (H) described in WO 2004/098542 contains at least one silane compound with at least 2 ethylenically unsaturated groups. Preferred silane compounds follow the general formula Si(R¹)ₙ(R²)₄₋ₙ, wherein R¹ is a linear, branched or cyclic monovalent ethylenically unsaturated substituent which can undergo an addition reaction with SiH-groups, having from 2 to 12 carbon atoms, R² is a monovalent radical without groups that can undergo an addition reaction with SiH-groups or have a detrimental influence on such a reaction with 1 to 12 carbon atoms and n is 2,3 or 4. Especially preferred radicals R¹ include vinyl, allyl and propargyl, especially preferred radicals R² include linear or branched C1-C12 alkyl groups.

The inventive composition further comprises as component (B) or part of component (B) a crosslinker compound containing at least two or three SiH groups.

By definition, an organohydrogenpolysiloxane according to the present text does not belong to the group of organopolysiloxanes used as component (A) or part of component (A) as described in the context of the invention.

An organohydrogenpolysiloxane according to the invention typically contains from 0.01 to 1.7 wt.-% silicon-bonded hydrogen or from 1.0 to 9.0 mmol SiH/g. The silicon valencies which are not saturated with hydrogen or oxygen atoms are typically saturated with monovalent hydrocarbon radicals R free from ethylenically unsaturated bonds.

The hydrocarbon radicals R, which may be selected independently from each other, represent a linear or branched or cyclic, non-substituted or substituted, aliphatic or aromatic monovalent hydrocarbon groups with 1 to 12 C atoms without ethylenically unsaturated bonds. In a preferred embodiment of the invention, at least 50%, preferably 100%, of the hydrocarbon radicals R that are bonded to silicon atoms are methyl radicals.

Organohydrogenpolysiloxanes which can be suitable as component (B) include those having a viscosity of 10 to 1,000 mPas or from 15 to 550 mPas or from 20 to 150 mPas.

The inventive composition also contains a catalyst as component (C) or as a part of component (C) capable of catalyzing a hydrosilation reaction.

This catalyst is typically a platinum catalyst or a platinum containing catalyst, including a platinum complex which can be prepared from hexachloroplatinum acid by reduction with tetramethyldivinyldisiloxane. Such compounds are known to the skilled person. Any other compounds which catalyze or accelerate addition cross-linking of silanes with ethylenically unsaturated double bonds are also suitable. Platinum-siloxane complexes as described, e.g. in US 3,715,334, US 3,775,352 and US 3,814,730 are suitable.

The catalyst can typically be used in an amount of 0.00005 to 0.05 wt.-%, particularly 0.0002 to 0.04 wt.-%, each calculated as elemental platinum and related to the overall weight of the composition.

Components (A), (B) and (C) are constituents of the hardenable matrix of the inventive composition.

The surfactants (D) can be summarized under the following formula 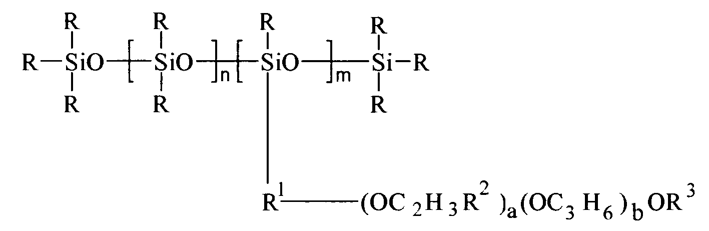 where each R is independently a monovalent hydrocarbyl radical with 1 to 22 C-atoms, R¹ is a divalent hydrocarbylene radical 1 to 26 C-atoms, each R² is independently hydrogen or a lower hydroxyalkyl radical, R³ is hydrogen or a monovalent hydrocarbyl radical with 1 to 22 C-atoms, n and b are independently greater than or equal to zero, and m and a are independently greater than or equal to one, with the proviso that a has a sufficient value and b is small enough so that a cured composition of the invention has the desired water contact angle.

Preferably R and R³ are -CH₃, R¹ is -C₃H₆-, R² is hydrogen, n is about zero or about one, m is about one to about five, a is about five to about 20 and b is about 0.

Several of such ethoxylated surfactants are for example available from Momentive Performance Materials Inc. including "SILWET" surface active copolymers. Preferred surface active copolymers include Silwet 35, Silwet L-77, Silwet L-7600 and Silwet L-7602 and Silwet Hydrostable 68 and Silwet Hydrostable 611. Silwet L-77 is an especially preferred ethoxylated surfactant which is believed to correspond to the above formula where R and R³ are -CH₃, R¹ is -C₃H₆-, R² is hydrogen, n is about zero or about one, m is about one or about two, a is about seven, and b is about 0. Also possible is the use of MASIL® SF19, as obtainable from Lubrizol performance products, Spartanburg, US.

Also possible is the use of polyether carbosilanes selected from the group consisting of:
Et₃Si-(CH₂)₃-O-(C₂H₄ O)y -CH₃, Et=Ethyl
Et₃Si-CH₂ -CH₂-O-(C₂ H₄O)y-CH₃, Et=Ethyl
(Me₃Si-CH₂)₃Si-(CH₂)₃-O-(C₂H₄O)y-CH₃, Me=Methyl
Me₃Si-CH₂-SiMe₂-(CH₂)₃-O-(C₂H₄O)y-CH₃, Me=Methyl
(Me₃Si-CH₂)₂SiMe-(CH₂)₃-O-(C₂H₄O)y-CH₃, Me=Methyl
Me₃Si-(CH₂)₃-O-(C₂H₄O)y-CH₃, Me=Methyl
Me₃Si-CH₂-CH₂-O-(C₂H₄O)y-CH₃, Me=Methyl
Ph₃Si-(CH₂)₃-O-(C₂H₄O)y-CH₃, Ph=phenyl
Ph₃Si-CH₂-CH₂-O-(C₂H₄O)y-CH₃, Ph=phenyl
Cy₃Si-(CH₂)₃-O-(C₂H₄O)y -CH₃, Cy=cyclohexyl
Cy₃Si-CH₂-CH₂-O-(C₂H₄O)y-CH₃, Cy=cyclohexyl
(C₆H₁₃)₃Si-(CH₂)₃-O-(C₂H₄O)y-CH₃
(C₆H₁₃)₃S₁-CH₂-CH₂-O-(C₄H₄O)y-CH₃ in which y conforms to the relation: 5 ≤ y ≤ 20.

Surfactants can be present in the inventive composition in an amount of more than 0.1 wt.-%, with respect to the weight of the whole composition. It can be preferred if the amount of component (D) is in a range of from 0.1 to 15 wt.-% or from 0.3 to 12 wt.-% or from 0.5 to 8 wt.-% or from 0.8 to 7 wt.-% or from 1 to 6 wt.-% or from 1.2 to 5 wt.-%. The composition of the invention is typically obtained by mixing a base paste and a catalyst paste. In this respect, the surfactant can be present in the base paste or the catalyst paste, or in the base paste and the catalyst paste. In one embodiment of the invention, the surfactant is present in the base paste only.

The F-containing compound(s) used in the composition of the present invention can be block-copolymers, alternating or statistic polymers, including regio-isomers, especially with regard to the HFPO (hexa fluor propylene oxide) unit.

The inventive composition comprises an F-containing compound having preferably a linear or branched backbone. The per- or partly fluorinated backbone of the F-containing compound is typically interrupted by one or more oxygen atoms.

Furthermore, certain embodiments of the F-containing compound can be characterized by the following features:
- Chain-length of the backbone: more than about 8 atoms and less than about 36 atoms (atoms counted along the longest chain in the molecule without taking into account the functional group T being selected from -COOR, -CH₂OH, -CF₂OR, - CHFOH, -CHFOR, -CH₂OR or -F with R being a linear or branched alkyl rest (C1 to C9), aryl rest (C1 to C9) or alkylaryl rest (C1 to C9).
- Containing 1 to about 10 or 2 to about 8 or 2 to about 6 ether structure elements.
- Containing at least one, two, three, four, five, six, seven or eight oxygen atom(s) connecting per- or partly fluorinated elements selected from CF₃-, -CHF-, -CF₂-, CF₃-CF₂-, -CF₂-CF₂-, -CHF-CF₂-, CF₃-CHF-, CF₃-CF₂-CF₂-, -CF₂-CF₂-CF₂-, - CF(CF₃)-CF₂-, -CF(CF₃)-, -CF₃-CF₂-CF₂-CF₂-, CF₃-CF₂-CF₂-CF₂-.
- The terminal groups in the molecule can be a perfluor or partly fluorinated linear or branched alkyl chain (e.g. C1-C6), a fluorine atom, an alcohol, an ether functionality or an ester functionality, wherein both terminal groups can be equal or different. The preferred esters or ethers are typically based on linear or branched C1-C9 alkyl chains, C1-C9 aryl residues or C1-C9 alkylaryl residues.
- The per- or partly fluorinated chain-segments of the main-chain do typically not comprise more than 5 atoms in a row.

The molecular structure of the F-containing compound does typically not comprise the following moieties: -SO₂NR- with R being a linear or branched alkyl group (e.g. C1 to C4), -OP(O)-O-, -N(R)-P(O)-O-, -N(R)-P(O)-NR- or metal ions.

The F-containing compound is preferably a low molecular compound with a molecular weight (Mn) equal or below 3000 g/mol or equal or below 1600 g/mol.

Typically, the molecular weight (Mn) is above 300 or above 350 or above 400. Thus, the molecular weight of the F-containing compound is typically within a range of 300 to 3000 or 350 to 2000 or 400 to 1600.

The F-containing compound is typically present in the curable dental composition in an amount effective to provide either a composition having a water contact angle of less than 20° or less than 13° at a water drop age of 10 s (Θ₁₀ₛ), determined according to the method described below 60 s after mixing of the components and/or an initial water contact angle (Θ₀ₛ) of less than 82°, 40 seconds after mixing of the components.

Typically, the F-containing compound is present in the curable composition in an amount of 0.1 to 5 wt.-% or 0.2 to 4 wt.-% with respect to the whole composition.

If the composition is provided as a kit of parts comprising a base paste and a catalyst paste, the F-containing compound can be present either in the base paste or the catalyst paste or in the base paste and the catalyst paste.

If the F-containing compound is present in the base paste, it is typically present in an amount of 0.1 to 6 wt.-% or 0.2 to 5 wt.-% or 0.5 to 4 wt.-% with respect to the whole weight of the base paste.

If the F-containing compound is present in the catalyst paste, it is typically present in an amount of 0.1 to 6 wt.-% or 0.2 to 0.5 wt.-% or 1 to 4 wt.-% with respect to the whole weight of the catalyst paste.

With respect to certain embodiments, it can be desirable if the F-containing compound is present in the catalyst paste only. This may contribute to providing a storage stable composition.

According to one embodiment of the invention, the molar ratio of F-containing compound to surfactant can be in a range of 0.1 to 4 or 0.25 to 2.4 or 0.3 to 1.8

According to another embodiment of the invention the fluor content of the F-containing compound can be in a range of 10 to 90 wt.-% or 40 to 70 wt.-% with respect to the molecular weight (Mn) of the F-containing compound.

Specific examples of the F-containing compound include:
- Rf-(O)ₜ-CHF-(CF₂)ₓ-T, with t = 0 or 1, x = 0 or 1 and Rf being a linear or branched per- or partly fluorinated alkyl rest (including C1 to C6 or C1 to C4), wherein the alkyl chain can be interrupted by O atoms,
- Rf-(OCF₂)ₘ-O-CF₂-T, with m = 1 to about 6 and Rf being a linear or branched per- or partly fluorinated alkyl rest (including C1 to C6 or C1 to C4), wherein the alkyl chain can be interrupted by O atoms,
- CF₃-(CF₂)₂-(OCF(CF₃)-CF₂)_{z}-O-L-T, with z = 0, 1, 2, 3, 4, 5 or 6, L having a structure selected from -CF(CF₃)-, -CF₂-, -CF₂CF₂- and -CHFCF₂,
- Rf-(O-CF₂CF₂)ₙ-O-CF₂-T, with n = 1, 2, 3, 4 or 5 and Rf being a linear or branched per- or partly fluorinated alkyl rest (including C1 to C6 or C1 to C4), wherein the alkyl chain can be interrupted by O atoms,
- an oligomeric compound obtainable by the anionic or photochemical (in the presence of oxygen) polymerization or copolymerisation of monomers selected from vinylidenfluoride, hexafluoropropylenoxide, tetrafluoroethylene, 2,2,3,3-tetrafluorooxetane, trifluoroethylene or monofluoroethylene, wherein at least one chain-end of the oligomeric compound is represented by a function T,
   Specific examples include
   a) homopolymerization of hexafluoropropylenoxide or 2,2,3,3-tetrafluorooxetane
   b) homopolymerization of vinylidenfluoride, hexafluoropropylenoxide, tetrafluoroethylene, 2,2,3,3-tetrafluorooxetane, trifluoroethylene or monofluoroethylene in the presence of oxygen
   T being selected from the group consisting of -COOR, -CH₂OR, -CHFOR, -CF₂OH, - CFHOH, -CH₂OH and mixtures thereof, with R being a linear or branched alkyl rest, aryl rest or alkylaryl rest (including C1 to C9).

In particular, the ester and in especially the methylester and the respective reduced alcohols or methylether of the following structures can be used. Specific examples of F-containing compounds, which can be used, include those listed below:
Rf-O-CHF-T, with Rf being a linear or branched per- or partly fluorinated alkyl chain (including C1 to C6), which can be interrupted by oxygen atoms.
Specific examples according to the above formula include:
CF₃-O-CF₂-O-CF₂-CF₂-O-CHF-T
CF₃-(O-CF₂)₂-O-CF₂-CF₂-O-CHF-T
CF₃-(O-CF₂)3-O-CF₂-CF₂-O-CHF-T

Rf-O-CHF-CF₂-T, with Rf being a linear or branched per- or partly fluorinated alkyl chain (including C1 to C6), which can be interrupted by oxygen atoms.
CF₃-O-CF₂-O-CF₂-CF₂-O-CHF-CF₂-T
CF₃-(O-CF₂)₂-O-CF₂-CF₂-O-CHF-CF₂-T
CF₃-(O-CF₂)₃-O-CF₂-CF₂-O-CHF-CF₂-T

R_{f}O-CF₂-CHF-T, with Rf being a linear or branched per- or partly fluorinated alkyl chain (including C1 to C6), which can be interrupted by oxygen atoms.
Specific examples according to the above formula include:
C₃F₇-O-CF₂-CHF-T
CF₃-O-CF₂-O-CF₂-CF₂-O-CF₂-CHF-T
CF₃-(O-CF₂)₂-O-CF₂-CF₂-O-CF₂-CHF-T
CF₃-(O-CF₂)₃-O-CF₂-CF₂-O-CF₂-CHF-T

Rf-O-CF₂-CHF-CF₂-T, with Rf being a linear or branched per- or partly fluorinated alkyl chain (including C1 to C6), which can be interrupted by oxygen atoms.
Specific examples according to the above formula include:
C₃F₇-O-CF₂-CHF-CF₂-T
CF₃-O-CF₂-CF₂-CF₂-O-CF₂-CHF-CF₂-T
CF₃-O-CF₂-O-CF₂-CF₂-O-CF₂-CHF-CF₂-T
CF₃-(O-CF₂)₂-O-CF₂-CF₂-O-CF₂-CHF-CF₂-T
CF₃-(O-CF₂)₃-O-CF₂-CF₂-O-CF₂-CHF-CF₂-T

R_{f}O-CF₂-CF₂-T, with Rf being a linear or branched per- or partly fluorinated alkyl chain (including C1 to C6), which can be interrupted by oxygen atoms, n = 1, 2 or 3 and m = 0 or 1.
Specific examples according to the above formula include:
CF₃-O-C F₂-CF₂-T
C₂F₅-O-CF₂-CF₂-T
C₃F₇-O-CF₂-CF₂-T
C₄F₉-O-CF₂-CF₂-T

Rf-(O-CF₂)ᵤ-O-CF₂-T, with Rf being a linear or branched per- or partly fluorinated alkyl chain (including C1 to C6), which can be interrupted by oxygen atoms, and u = 1, 2, 3, 4, 5 or 6.
Specific examples according to the above formula include:
CF₃-(O-CF₂)₃-O-CF₂-T
CF₃-(O-CF₂)₅-O-CF₂-T

Rf-(O-CF₂-CF₂)ₖ-O-CF₂-T , with Rf being a linear or branched per- or partly fluorinated alkyl chain (including C1 to C6), which can be interrupted by oxygen atoms and k = 1, 2, 3,4,5.
C₂F₅-(O-CF₂-CF₂)₁-O-CF₂-T
C₃F₇(O-CF₂-CF₂)₁O-CF₂-T
C₄F₉-(O-CF₂-CF₂)₁-O-CF₂-T
C₂F₅-(O-CF₂-CF₂)₂-O-CF₂-T
CF₃-(O-CF₂-CF₂)₂-O-CF₂-T
C₃F₇-(O-CF₂-CF₂)₂-O-CF₂-T
C₄F₉-(O-CF₂-CF₂)₂-O-CF₂-T

Rf-O-CF₂-T, with Rf being a linear or branched per- or partly fluorinated alkyl chain (including C1 to C6), which can be interrupted by oxygen atoms.
Specific examples according to the above formula include:
C₃F₇-O-CF₂-T

CF₃-(CF₂)₂-(O-CF(CF₃)-CF₂)_{z}-O-CF(CF₃)-T with z = 0, 1, 2, 3, 4, 5, 6, 7 or 8,
Specific examples according to the above formula include:
CF₃-(CF₂)₂-(O-CF(CF₃)-CF₂)₂-O-CF(CF₃)-T
CF₃-(CF₂)₂-(O-CF(CF₃)-CF₂)₃-O-CF(CF₃)-T
CF₃-(CF₂)₂-(O-CF(CF₃)-CF₂)₄-O-CF(CF₃)-T
CF₃-(CF₂)₂-(O-CF(CF₃)-CF₂)₅-O-CF(CF₃)-T

CF₃-CHF-O-(CF₂)ₒ-T, with o = 1, 2, 3, 4, 5 or 6.
Specific examples according to the above formula include:
CF₃-CFH-O-(CF₂)₃-T
CF₃-CFH-O-(CF₂)₅-T

CF₃-CF₂-O-(CF₂)ₒ-T, with o = 1, 2, 3, 4, 5 or 6.
Specific examples according to the above formula include:
CF₃-CF₂-O-(CF₂)₃-T
CF₃-CF₂-O-(CF₂)₅-T.

T-CF₂-O-(CF₂-CF₂-O)ₚ-(CF₂-O)_{q}-CF₂-T , with p/q = about 0.5 to about 3.0 and an molecular weight in the range of about 500 to about 4000 g/mol.

T-CF₂-(O-CF(CF₃)-CF₂)ₙ- (O-CF₂)ₘ-O-CF₂-T with n/m = about 20 to about 40 and a molecular weight in the range of about 650 to about 3200 g/mol.

Rf-(O-CF₂-CF₂-CF₂)ₙ-O-CF₂-CF₂-T with n = 1-25 and Rf being a linear or branched per- or partly fluorinated alkyl chain (including C1 to C6), wherein the alkyl chain can be interrupted by O atoms.

In the above formulas T is selected from the group consisting of -COOR, -CH₂OR, - CHFOR, -CF₂OH, -CFHOH, -CH₂OH or -F and mixtures thereof, with R being a linear or branched alkyl rest (including C1 to C9), aryl rest (including C1 to C9) or alkylaryl rest (including C1 to C9).

In another specific embodiment of the invention, the curable dental composition comprises at least two different F-containing components. It has been found, that a mixture of different F-containing components (in addition to a surfactant) might even further improve the wetting behaviour.

A very specific example includes a mixture of HFPO and 1H,1H-perfluor-3,6,9-trioxatridecan-1-ol.

HFPO (Hexa fluoro propylene oxide) can be obtained as described in US 3,242,218 or US 2004/0124396. The general formula of HFPO is C₃F₇O[CF(CF₃)CF₂O]ₙCF(CF₃)COOCH₃ with n being 1 to 6.

According to another embodiment, the inventive composition may contain a filler or a mixture of fillers, e.g. as component (F) or as a part of component (F), even if the presence of a filler is not mandatory. The nature of the filler is not particularly limited.

Typically filler can be used in an amount of from of at least 5 wt.-% or at least 20 or at least 35 wt.-% with respect to the whole composition.

There is no particular upper limit, however, typically the amount of filler, if present at all, is used in an amount of at most 80 wt.-% or at most 70 wt.-% or at most 50 wt.-% with respect to the whole composition.

Thus, typical ranges for the filler as component (F) include from 5 to 80 or from 20 to 70 or from 35 to 50 wt.-% with respect to the whole composition.

A wide variety of inorganic, hydrophilic or hydrophobic fillers may be employed such as silicas, aluminas, magnesias, titanias, inorganic salts, metallic oxides and glasses. It has been found to be possible to employ mixtures of silicone dioxides, including those derived from crystalline silicone dioxide, such as pulverized quartz (4-6 µm); amorphous silicone dioxides, such as a diatomaceous earth (4-7 µm); and silanated fumed silica, such as Cab-o-Sil TS-530 (160-240 m²/g), manufactured by Cabot Corporation.

The sizes and surface areas of the foregoing materials can be adjusted to control the viscosity and thixotropicity of the resulting compositions. Some or all of the foregoing hydrophobic fillers may be superficially treated with one or more silanating agents, as known to those of ordinary skill in the art. Such silanating may be accomplished through use of known halogenated silanes or alkoxysilanes or silazanes. Such fillers can be present in amounts of from 20 to 80 % by weight, especially 25 to 70 or 30 to 60 wt.-% of the material.

Among the fillers which can be used are fillers such as quartz, cristobalite, calcium silicate, diatomaceous earth, zirconium silicate, montmorillonite such as bentonite, zeolite, including molecular sieves such as sodium aluminium silicate, metal oxide powder such as aluminium or zinc oxide or their mixed oxides, barium sulphate, calcium carbonate, plaster, glass and plastic powder.

Suitable fillers are also pyrogenic or precipitated silicic acid and silica aluminium mixed oxides. Those filler are commercially available from companies like Wacker or Degussa under the trade names Aerosil^{™}, HDK-H.

The above mentioned fillers can be hydrophobized, for example by treatment with organosilanes or siloxanes or by the etherification of hydroxyl groups to alkoxy groups. One type of filler or also a mixture of at least two fillers can be used. The particle distribution is preferably chosen such that there are no fillers with particle sizes of more than about 50 µm.

A combination of reinforcing and non-reinforcing fillers can be preferred. In this respect, the quantity of reinforcing fillers can range from 0.1 to 10 wt.-%, in particular from 2 to 7 wt.-% with respect to the whole composition.

Typical reinforcing fillers include fumed silica, carbon black and the like. They also can be surface treated and can improve mechanical properties like tensile strength or tear strength, of the cured silicone composition.

Pyrogenically-prepared highly-disperse silicic acids which have preferably been hydrophobized by surface treatment are preferred as reinforcing fillers. The surface treatment can be carried out, for example with dimethyldichlorosilane, hexamethyldisilazane, tetramethylcyclotetrasiloxane or polymethylsiloxane.

Preferred non-reinforcing fillers are quartzes, cristobalites and sodium aluminium silicates which can be surface-treated. The surface treatment can generally be carried out with the same methods as described in the case of the strengthening fillers.

Typical non-reinforcing fillers are quartz, precipitated silicas, diatomaceous earth, aluminas, magnesias, titanium dioxide, zirconium silicate, metallic oxides, and the like. These fillers can be surface treated, e.g. silanated, or non surface treated. Typical particle sizes are 2 to 10 µm.

According to another embodiment, the curable dental composition of the invention may contain organopolysiloxanes without reactive substituents as component (G) or part of component (G) even if the presence of such a component is not mandatory.

Non-reactive substituents include those which do not co-polymerize with the other components of the composition during the hardening process. These are preferably linear, branched or cyclic organopolysiloxanes where all silicon atoms are surrounded by oxygen atoms or monovalent hydrocarbon radicals with 1 to18 carbon atoms which can be substituted or non-substituted. The hydrocarbon radicals can be methyl, ethyl, C3-C10 aliphatics, trifluoropropyl groups as well as aromatic C6-C12 radicals.

Polydimethylsiloxanes with trimethylsiloxy end groups are particularly preferred as a constituent of component (G). Component (G) can be used in an amount of 0 to 40 wt.-%, or 0.1 to 20 wt.-% or 0.5 to 15 wt.-%.

According to a further embodiment, the inventive composition can also contain other additives e.g. as component (H) or part of component (H).

Those additives include retarders to modify the working and setting time (e.g. 3-methyl-I-butyne-3-ol or 1,1,3,3-tetramethyl-1,3-divinyl siloxane (VMO)), rheology modifiers (e.g. synthetic or natural waxes or polyethylene/propylene diacetats as described in EP 1 165 016 A1; corresponding to US 6,677,393), inhibitors, pigments, dyes, plastizers (including paraffin oil or mineral oil), odorous substances, flavourings, stabilizers (including diphosphite(s) as described e.g. in WO 2007/001896 A2) or hydrogen scavenger etc. alone or in admixture.

The additive(s) can be present in an amount in the range of 0.01 to 90 % by weight, or in the range of 0.1 to 40 % by weight with respect to the cured composition.

To control the reactivity of the addition reaction and to prevent premature curing, it may be advantageous to add an inhibitor, which prevents the addition reaction for a specific period of time or slows the addition reaction down. Such inhibitors are known and described, e.g. in US 3,933,880. Examples of such inhibitors include acetylenic unsaturated alcohols such as 3-methyl-I-butyne-3-ol, 1-ethynylcyclohexane-I-ol, 3,5-dimethyl-I-hexyne-3-ol and 3-methyl-I-pentyne-3-ol. Examples of inhibitors based an vinyl siloxane are 1,1,3,3-tetramethyl-1,3-divinyl siloxane, 1,3,5,7-tetravinyl-1,3,5,7-tetramethylcyclotetrasiloxane and poly-, oligo- and disiloxanes containing vinyl groups.

The composition may also contain a component useful for diminishing the presence or degree of hydrogen outgassing which may be typically generated as a result of the vinyl polymerization. The composition thus may comprise a hydrogen scavenger such as finely divided platinum metal that scavenges for and takes up such hydrogen. The Pt metal may be deposited upon a substantially insoluble salt having a surface area of between about 0.1 and about 40m²/g. Suitable salts are Barium sulphate, barium carbonate and calcium carbonate of suitable particle sizes. Other substrates include diatomaceous earth, activated alumina, activated carbon and others. The inorganic salts are especially preferred to imply improved stability to the resulting materials incorporating them. Dispersed upon the salts is about 0.2 to about 2 parts per million of platinum metal, based upon the weight of the catalyst component. It has been found that employment of the platinum metal dispersed upon inorganic salt particles substantially eliminates or diminishes hydrogen outgassing during curing of dental silicones. Also Pd metal as described e.g. in US 4,273,902 or Pd compounds as disclosed in to US 5,684,060 can be employed.

The curable composition of the invention can also contain a polyethylene glycol derivate, (including polyethylene glycol dimethylether) having a chain-length e.g. equal or below about 1000 g/mol.

According to one embodiment of the invention, the composition can comprise the individual components in the following amounts:
Component (A): from 20 wt.-% to 60 wt.-% or from 25 wt.-% to 55 wt.-% or from 36 wt.-% to 53 wt.-% with respect to the whole composition.
Component (B): from 0.1 wt.-% to 15 wt.-% or from 1 wt.-% to 10 wt.-% or from 3 wt.-% to 5 wt.-% with respect to the whole composition.
Component (C): from 0.001 wt.-% to 0.1 wt.-% or from 0.002 wt.-% to 0.02 wt.-% or from 0.005 wt.-% to 0.01 wt.-% with respect to the whole composition.
Component (D): from 0.1 wt.-% to 60 wt.-% or from 0.3 wt.-% to 55 wt.-% or from 0.5 wt.-% to 50 wt.-% with respect to the whole composition.
Component (E): from 0 wt.-% to 30 wt.-% or from 0.3 wt.-% to 25 wt.-% or from 0.5 wt.-% to 20 wt.-% with respect to the whole composition.
Component (F): from 0 wt.-% to 80 wt.-% or from 0.1 wt.-% to 70 wt.-% or from 0.5 wt.-% to 50 wt.-% with respect to the whole composition.
Component (G): from 0 wt.-% to 40 wt.-% or from 0 wt.-% to 30 wt.-% or from 0 wt.-% to 20 wt.-% with respect to the whole composition.
Component (H): from 0 wt.-% to 90 wt.-% or from 0.01 wt.-% to 40 wt with respect to the whole composition.

The dental compositions according to the invention are typically multi component materials which comprise at least a curable base paste and a catalyst paste comprising a catalyst for curing at least part of the material of the base paste.

Accordingly, the components of the composition can be included in a kit, where the contents of the composition are packaged to allow for storage of the components until they are needed. When used, the components of the compositions can be mixed in the suitable amounts and clinically applied using conventional techniques.

Thus, the invention also relates to a kit of parts, comprising a base paste and a catalyst pate separated from each other before use, wherein the base paste comprises components (A) and (B), and the catalyst paste comprises component (C) or (C) and (A), and wherein component (D) is present either in the base paste or the catalyst paste or in the base paste and the catalyst paste. The same applies to component (E). The other optional components (F), (G), and (H) can be present in the base paste or the catalyst paste or in the base paste and the catalyst paste.

According to a certain embodiment of the invention, the surfactant is present in the base paste and the F-containing compound is present in the catalyst paste.

According to another embodiment of the invention, the base paste is essentially free of the F-containing compound and the catalyst paste is essentially free of the surfactant.

The volume ratios of catalyst paste and base paste can range from about 10:1 to about 1:10. Particularly preferred volume ratios of base paste to catalyst paste are about 1:1 and about 5:1 (5 parts of base paste to 1 part of catalyst paste).

Generally, mixing and dosing of the components can be performed manually, e.g., by spatula (strand-length comparison) or a manually operated pre-filled dual cartridge dispenser with static mixing tips, or automated, using one of the various available devices available for such an automated task, preferably one of the devices mentioned in EP 0 232 733 A1, US 5,924,600, US 6,135,631 or EP 0 863 088 A1 together with a dynamic mixing tip as mentioned in US 2004/0085854 or US 6,244,740.

A further improvement of the handling properties of dental compositions can be seen in using an automatic mixing and metering systems for two-component compositions which have automatic conveying and mixing units, such as are described e.g. in US 5,249,862, US 5,286,105 and US 5,332,122. The need for manual mixing of base pastes and catalyst pastes, above all when mixing larger quantities of material, can be eliminated, since this can take place automatically and within a short period of time. The result is usually a homogeneous product which is essentially free of air bubbles. Commercially available devices are distributed by 3M ESPE under the brand Pentamix^{™} or Pentamix^{™} 2.

In practice, the impression material can be syringed through a static or mechanical mixing device into an impression tray or onto patient's teeth or tissue and placed in the patient's mouth. After the impression material is set, the tray is removed from the patient's mouth and, in instances where the dental practitioner prepares the positive model, it may be preferable to pour the positive model material immediately after removal of the impression from the patient's mouth.

The invention also relates to a method of producing a curable composition comprising the step of combining an F-containing compound with a hardenable matrix or a composition comprising components (A), (B), (C), wherein components (A), (B) and (C) are as described in the text of the invention.

Typically, after combining of the F-containing compound with the hardenable matrix or the individual components of the hardenable matrix, the components are mixed. The F-containing compound can be added to the other components of the composition from the very beginning of the production process or during or at the end of the production process. The F-containing compound can also be applied as a pre-mixture with component (D).

The dental material or composition can be used as impression material or for the production of (temporary or long term) crown and/or bridges. In the latter case, the inventive composition is used as a mould to be filled with the (temporary or long term) crown and/or bridge material, which is typically based on polymerizable (meth)acrylates.

Another aspect of the invention, relates to a method of using the F-containing compound as described in the text of the invention for enhancing the hydrophilicity of a hardenable composition comprising a surfactant. The method typically includes the step of adding to or combining the F-containing compound with a hardenable matrix.

Features and advantages of this invention are further illustrated by the following examples, which are in no way intended to be limiting thereof. The particular materials and amounts thereof recited in these examples, as well as other conditions and details, should not be construed to unduly limit this invention. Unless otherwise indicated, all parts and percentages are on a weight basis, all water is deionized water, all molecular weights are weight average molecular weight and all measurements were done at ambient conditions (23°C).

### Examples

### Measurements

### Water contact angle measurement of un-cured paste

Test specimen preparation: For the preparation of test piece the mixed paste was subjected to an object slide and flattened and triturated by a second object slide in order to obtain a thin film. The test piece preparation was performed in that simplified way as the thickness of the film does not have a significant effect on the measured water contact angle (see G. Kugel, T. Klettke, J. A. Goldberg, J. Benchimol, R. D. Perry, S. Sharma, J Prosthod. 2007, 16, 84-92). Measurement: The object slide was placed on the table of a Drop Shape Analyse System DSA 10 (Krüss GmbH, Hamburg), a well known device for measuring contact angles. 5 µl of water were placed onto the surface of the specimen and an automatic contact angle measurement was started using standard software of the goniometer. Measuring time was at least about 10 s up to about 200 s. The water contact angle was measured at different time periods after mixing of base paste and catalyst paste, especially after 40 and 60 s. The data (video sequences) were evaluated by the "circle fitting" method, another standard method for data evaluation (see G. Kugel, T. Klettke, J. A. Goldberg, J. Benchimol, R. D. Perry, S. Sharma, J. Prosthod. 2007, 16, 84-92).
Θ ₁₀ₛ is the angle obtained 10 s after placing the water drop on the surface.
Θ ₀ₛ is the angle obtained immediately after placing the water drop on the surface (initial water contact angle).

### Determination of setting time

The setting time of the compositions was determined by measuring the viscosity in dependence on the time at 33 °C by using a MDR 2000 rheometer from Alpha instruments under aerobic conditions at 50% humidity. The setting time was determined as the t₉₀ value, at which 90% of the final viscosity was achieved. Another characteristic size is the t₅ value, at which 5% of the final viscosity was present. Until this time the composition can be assumed to be almost free of network formation (curing).

### Tensile strength and elongation at break

The tensile strength and elongation of the compositions was determined according to DIN 53504. The tensile strength is given in MPa and the elongation in % of the original length. Tensile strength and elongation data were evaluated by tearing six I-shaped specimens with a central unit of 20 mm x 4 mm x 2 mm in a Zwick Z020 Universal testing machine. Base and catalyst pastes were mixed through a static mixer (SulzerMixpac Comp.) and filled into a brass mould. After 3 h at 23 °C the specimen were removed, six measurements were made and the mean value determined (speed 200 mm/min).

### Linear dimensional change

The linear dimensional change of the compositions was determined according to ISO 4823 and is given in %.

### Viscosity

The viscosity was measured at 23 °C using a Haake Rotovisco 1 device with a plate/plate system (diameter 20 mm) and a slit of 0.2 mm. The viscosity values (Pas) and share stress values (Pa) are recorded for each share rate ( . starting from 10 1/s to 100 1/s in 10 1/s and/or 5 1/s steps. For each share rate, a delay of 5 seconds was used before collecting data. The above mentioned method of measurement corresponds essentially to DIN 53018-1.

### Recovery from elongation

The recovery from elongation of the compositions was determined and is given in %. The recovery from elongation was evaluated by tearing six I-shaped specimens with a central unit of 20 mm x 4 mm x 2 mm (additionally comprising two markers on each side of the test specimen in a distance of 20 mm) in a Zwick Z020 Universal testing machine. Base and catalyst pastes were mixed through a static mixer (SulzerMixpac Comp.) and filled into a brass mould placed on a foil. The mold is covered with another foil and pressed with a glass plate. 25 seconds after mixing of base and catalyst paste, the mold is placed in 35°C water bath and is cured in the water bath (according to the setting time). The mold is taken out of the water bath and dried. Exactly 1 min and 30 s after removal out of the water bath the test specimen is elongated to 150% of its original length, held for 2 s and relaxed.

After storing for 2 hours at 23°C and 50% humidity, the length between the markers was measured. For control a test body without elongation was measured.

### Shelf life time determination

For the determination of the shelf life time, the catalyst paste B containing 1.5 wt.-% of HFPO, 1H,1H-Perfluor-3,6,9-trioxatridecan-1-ol, Silwet L-77 or Zonyl FSO-100, respectively, was filled in a conventional foil bag (3M ESPE) and stored at 70°C. After the storage time given in Table 8 below, the setting time was determined according to the method described above by mixing the stored catalyst paste with an at room temperature stored base paste (according to base paste formula A, see below).

### General Description - Preparation of Composition

The base and the catalyst paste used hereafter have been prepared in a vacuum kneader by mixing the respective components to a homogenous paste.

### Base paste formula A:

| | |
|---|---|
| Mixture of vinyl terminated polydimethylsiloxane (7,900 mPas) | 50.5% (weight) |
| Poly(methyl)(hydrogen)siloxane (40 to 160 mPas) | 11.9% (weight) |
| Pyrogenic silica (hydrophobized, 100 m²/g) | 5.9% (weight) |
| Crystalline SiO₂ filler (< 20 µm) | 31.7% (weight) |

### Catalyst paste formula A (regular setting material):

| | |
|---|---|
| Mixture of vinyl terminated polydimethylsiloxane (5,800 mPas) | 48.6% (weight) |
| Tetraallylsilane | 0.5% (weight) |
| Platin tetramethyldivinyldisiloxane (VMO) complex 1.3 wt.-% Pt in silicone oil | 0.2% (weight) |
| Platin tetramethyldivinyldisiloxane complex 1.3 wt.-% Pt in silicone oil containing in addition 4 % of VMO | 1.4% (weight) |
| Palladium chloride dispersion in vinyl terminated polydimethylsiloxane (2,000 mPas) | 0.1% (weight) |
| Pyrogenic silica (hydrophobized, 100 m²/g) | 5.7% (weight) |
| Crystalline SiO₂ filler (< 20 µm) | 42.7% (weight) |
| Pigment dispersion in polydimethylsiloxane (30,000 to 100,000mPas) | 0.8% (weight) |

### Catalyst paste formula B (fast setting material):

Catalyst paste formula B differs from catalyst paste formula A with respect to the platinum complex. The other components were the same.

| | |
|---|---|
| Platin tetramethyldivinyldisiloxane (VMO) complex 1.3 wt.-% Pt in silicone oil containing in addition 0.3% VMO | 1.12% (weight) |
| Platin tetramethyldivinyldisiloxane complex 1.3 wt.-% Pt in silicone oil containing in addition 4 % of VMO | 0.48% (weight) |

X wt.-% of surfactant and Y wt.-% of the F-containing compound were added to the base paste A and/or the catalyst pastes A or B, respectively, and kneaded under vacuum for about 30 min to obtain a homogenous paste (Table 1).

The base paste and catalyst paste were filled in a dual chamber cartridge (SulzerMixpac Comp.), volume ratio 1:1, equipped with a static mixing tip (SulzerMixpac Company). The pastes were extruded from the cartridge and mixed using a hand mixing apparatus (3M ESPE Comp.).

The un-cured and cured compositions were tested with respect to their wetting behaviour (Tables 2, 3, 4, 5 and 8) and the cured compositions with respect to their physical parameters (Table 6). The storage stability of the catalyst paste containing 1.5 wt.-% of surfactant or F-containing compound was tested and the results are summarized in Table 7.

**Table 1**

| regular setting material t_{5/}t₉₀ =2.03 / 3.56 [min] | Base paste A | | Catalyste paste A | | |
|---|---|---|---|---|---|
| | F-containing compound | surfactant | F- containing compound | surfactant | F-containing compound, surfactant used |
| Example 1 | 3.5 | 3.0 | - | - | HFPO^{c)}, Silwet L-77^{a)} |
| Example 2 | 3.5 | 3.0 | - | 1,5 | HFPO^{c)}, Silwet L-77^{a)} |
| Example 3 | 3.5 | 3.0 | 1,5 | - | HFPO^{c)}, Silwet L-77^{a)} |
| Example 4 | 3.5 | 3.0 | 1,5 | - | HFPO^{c)}, Silwet L-77^{a)} |
| Example 5 | 3.5 | 3.0 | - | - | HFPO ^{c)}, Carbosilantensid^{b)} |
| C. Example V1 | - | 3.0 | - | - | Silwet L-77 ^{a)} |
| C. Example V2 | - | 3.0 | - | - | Carbosilantensid^{b)} |
| C. Example V3 | 3.5 | - | - | - | HFPO^{c)} |
| C. Example V4 | 3.5 | - | - | - | Zonyl FSO-100^{e)} |
| Fast setting material t₅/t₉₀ =1.48 / 2.48 [min] | Base Paste A | | Catalyst Paste B | | |
| Example 6 | 3.5 | 3.0 | - | - | HFPO ^{c)}, silwet L-77^{a)} |
| Example 7 | 3.5 | 3.0 | 1.5 | | HFPO^{c)} Silwet L-77^{a)} Silwet L-77^{a)} |
| Example 8 | 3.5 | 3.0 | - | - | 1H,1H-Perfluor-3,6,9-trioxa-3,6,9-trioxa- ^{d)}, tridecan-1-ol , Silwet L-77 ^{a)} |
| Example 9 | 2.0 | 3.0 | - | - | 1H,1H-Perfluor-3,6,9-trioxa-tridecan-1-ol ^{d)}, Silwet L-77 ^{a)} |
| Example 10 | 5.0 | 3.0 | - | - | 1H,1H-Perfluor-3,6,9-trioxa-tridecan-1-ol ^{d)}, Silwet L-77 ^{a)} |
| Example 11 | - | 3.0 | 1.5 | - | 1H,1H-Perfluor-3,6,9-trioxa- ^{d)}, tridecan-1-ol ^{d)}, Silwet L-77 ^{a)} |
| Example 12 | - | 3.0 | 3.5 | - | 1H,1H-Perfluor-3,6,9-trioxa- ^{d)}, 1 tridecan-1-ol ^{d)}, Silwet L-77^{a)} |
| Example 13 | 2.0 | 3.0 | 1.5 | - | 1H,1H-Perfluor-3,6,9-trioxa-tridecan-1-ol^{d)}, Silwet L-77^{a)} |
| C. Example V5 | 3.5 | 3.0 | - | - | Zonyl FSO-100^{e)}, Silwet L-77^{a)} |
| C. Example V6 | - | 3.0 | - | - | Silwet-L77^{a)} |

| | | | | | |
|---|---|---|---|---|---|
| C. Example means comparative example. ^{a)} Silwet L-77 (Momentive Corp.) ^{b)} Carbosilantenside can be obtained as described in Production Example 2 in US 5,750,589. ^{c)} HFPO (hexa fluor propylene oxide) oligomers: Dyneon LCC ^{d)} 1 H,1H-Perfluor-3,6,9-trioxatridecan-1-ol is obtainable from Apollo Scientific Ltd. ^{e)} Zonyl FSO-100: ethoxylated perfluoroalcanol (obtainable from Aldrich or Du Pont) having an HLB-value of 11.5 according to WO 2007/080071 A2 and technical data sheet. | | | | | |

Table 2 shows the water contact angles 40 s and 60 s after mixing of base paste and catalyst paste for the Examples given in Table 1 (measured according to the description above with respect to Water contact measurement of un-cured paste).

**Table 2**

| | Water contact angle Θ₁₀ₛ (°); 40 s after mixing | Water contact angle Θ₁₀ₛ (°); 60 s after mixing |
|---|---|---|
| Example 1 | 40 | <2 |
| Example 2 | 2 | 3 |
| Example 3 | 76 | <5 |
| Example 4 | 3 | 3 |
| Example 5 | 49 | 12 |
| C. Example V1 | 70 | 70 |
| C. Example V2 | 60 | 57 |
| C. Example V3 | 96 | 97 |
| C. Example V4 | 83 | 65 |
| Example 6 | 6 | 2 |
| Example 7 | 70 | <6 |
| Example 8 | 10 | 7 |
| Example 9 | 51 | 51 |
| Example 10 | 60 | 65 |
| Example 11 | 44 | 51 |
| Example 12 | 59 | 71 |
| Example 13 | 57 | 67 |
| C. Example V5 | 4 | 2 |
| C. Example V6 | 60 | 56 |

Another feature determined was the time a water drop needs to reach a water contact angle of 10° after mixing the composition (t ( 10°) in s). This time mirrors the clinical situation and reflects the ability to wet the saliva fast and efficient and, thus, to realize an effective water extrusion and provide sufficient detail accuracy. The time that a drop needs to reach a water contact angle of 10° at different times after mixing of base paste and catalyst paste is summarized in Table 3.

**Table 3**

| | 40 s | 60 s |
|---|---|---|
| Example 1 | 17 | 5 |
| Example 2 | 2 | 3 |
| Example 3 | 13 | 4 |
| Example 4 | 2 | 1 |
| Example 5 | n.d.^{a)} | 10 |
| C. Example V1 | n.d.^{a)} | n.d.^{a)} |
| C. Example V2 | n.d.^{a)} | n.d.^{a)} |
| C. Example V3 | n.d.^{a)} | n.d.^{a)} |
| C. Example V4 | n.d.^{a)} | n.d.^{a)} |
| Example 6 | 9 | 4 |
| Example 7 | 18 | 2 |
| Example 8 | 4 | 3 |
| Example 9 | n.d.^{a)} | n.d.^{a)} |
| Example 10 | n.d. ^{a)} | n.d.^{a)} |
| Example 11 | n.d.^{a)} | n.d.^{a)} |
| Example 12 | n.d.^{a)} | n.d.^{a)} |
| Example 13 | n.d. ^{a)} | n.d.^{a)} |
| C. Example V5 | 3 | 2 |
| C. Example V6 | n.d. ^{a)} | n.d.^{a)} |

| | | |
|---|---|---|
| ^{a)} water contact angle during measurement time always above 10° | | |

The initial contact angles ( ₀ₛ in degrees) obtained for Examples 8-13 are summarized in Table 4 and the initial contact angle of Example 13 in dependence on the time after mixing is summarized in Table 5.

**Table 4**

| | 40 s | 60 s |
|---|---|---|
| Example 8 | 70° | 65° |
| Example 9 | 63° | 57° |
| Example 10 | 67 | 73 |
| Example 11 | 52° | 57° |
| Example 12 | 82° | 83° |
| Example 13 | 73° | 75° |

**Table 5**

| Time after mixing in s | ₀ₛ |
|---|---|
| 12 | 76° |
| 20 | 75° |
| 40 | 73° |
| 60 | 75° |

A comparison of other physical parameters of exemplified cured composition containing either 1.75 wt.-% HFPO (Example 6) or Zonyl FSO-100 (Comparative Example 5) and 1.5 wt.-% Silwet L-77 is shown in Table 6.

**Table 6**

| | Example 6 | C. Example 5 |
|---|---|---|
| Tensile strength [MPa] | 4.76 | 4.50 |
| Elongation [%] | 262 | 242 |
| Recovery from elongation [%] | 0.023 | 0.081 |
| viscosity base paste [Pas]; = [50 1/s] | 17.219 | 18.276 |

### For comparison:

Viscosity of base paste A (without containing Silwett L-77): 23.558 Pas
Viscosity of base paste A containing 3.0 wt.-% Silwet L-77: 18.800 Pas
Viscosity of composition of Example 15 (containing Silwett L-77, HFPO and 1 H,1 H-Perfluor-3,6,9-trioxatridecan-1-ol): 15.604 Pas

As can be seen from Table 6, the addition of HFPO (instead of Zonyl FSO-100) leads to a composition having a lower viscosity (i.e. a better flowing behavior) without jeopardizing tensile strength and elongation at break.

In Table 7 the shelf life times of catalyst paste B containing 1.5 wt.-% of surfactant or F-containing compound at 70 °C are summarized. The values indicate the working time/setting time [min] after storing the composition at 70°C for various days.

**Table 7**

| T = 70 °C | Start | 2 d | 4 d | 7 d | 14 d | 21 d | 28d | 35d | 42d |
|---|---|---|---|---|---|---|---|---|---|
| 1H,1H-Perfluoro-3,6,9-trioxatridecan-1-ol | 0.93/ 2.16 | -- | -- | 1.04/ 2.41 | 1.01/ 2.31 | 5.31/ 9.08 | 6.65/ 9.84 | 3.01/ 6.81 | 4.11/ 5.82 |
| HFPO | 1.20/ 2.79 | 1.41/ 2.79 | 5.65 />10 | No set | No set | No set | No set | No set | No set |
| Zonyl FSO-100 | 1.74/ 5.46 | No set | No set | No set | No set | No set | No set | No set | No set |
| Silwet L-77 | 0.94/ 2.34 | 1.46/ 3.09 | 2.67/ 5.74 | No set | No set | No set | No set | No set | No set |
| Catalyst paste B | 1.48/ 2.48 | -- | -- | 1.15/ 2.18 | 6.28/ 9.54 | No set | No set | No set | No set |

### Example 14:

Base paste A and catalyst paste B were mixed according to the general procedure described above with 3.0 wt-% Silwet L-77 and 2.0 wt.-% of HFPO and 1.5% of 1H,1H-perfluor-3,6,9-trioxatridecan-1-ol The wetting behaviour was determined as described above 30, 40 and 60 seconds after mixing of base paste and catalyst paste.

### Example 15:

Base paste A and catalyst paste B were mixed according to the general procedure described above with 3.0 wt-% Silwet L-77 and 2.5 wt.-% of HFPO and 1.0% of 1 H, 1 H-perfluor-3,6,9-trioxatridecan-1-ol. The wetting behaviour was determined as described above 20, 30 and 40 seconds after mixing of base paste and catalyst paste. The results summarized in Table 8.

The initial contact angles and the water contact angles as well as the time a water drop needs to reach a contact angle of 10° are summarized in Table 8. These results reveal that using a mixture of different F-containing compounds might even improve the wetting behaviour of the curable composition.

**Table 8**

| | 20 seconds | | | 30 seconds | | 40 seconds | | | 60 seconds | |
|---|---|---|---|---|---|---|---|---|---|---|
| Example | C.E.5 | 14 | 15 | 14 | 15 | C.E.5 | 14 | 15 | 14 | 15 |
| ₀ₛ in degree | 109 | - | 27 | 68 | 30 | 67 | 56 | 36 | 25 | - |
| ₁₀ₛ in degree | 7 | - | 6 | 48 | 6 | 4 | 46 | 5 | 6 | - |
| t ( 10°) in s | 6 | - | 4 | n.d. | 4 | 4 | n.d. | 3 | 4 | - |

Fig.1 shows the time dependency of the water contact angle of exemplified compositions measured at different time periods after mixing of base paste and catalyst paste.

## Claims

1. A curable dental composition comprising
a. a curable silicone polymer as component (A), the silicone polymer containing at least two functional groups capable of reacting with a SiH group in the presence of a hydrosilation catalyst,
b. a crosslinker compound containing at least two SiH groups as component (B),
c. a catalyst capable of catalyzing a hydrosilation reaction as component (C),
d. a surfactant as component (D).
e. an F-containing compound as component (E), wherein the F-containing compound has the following formula
T₁-X-[(O-CF₂-CF₂)ᵤ-(O-CF₂)ᵥ-(O-CF(CF₃)-CF₂)_{w}-(O-CF₂-CF₂-CF₂)ₓ-O]-X-T₂ with u = 0 to 8, v = 0 to 8, w = 0 to 8 and x =0 to 8 and u+v+w+x ≥ 1,
wherein T₁ and T₂ can be equal or different and are independently selected from -COOR, -CH₂OH, -CF₂OR, -CHFOH, -CHFOR, -CH₂OR or -F with R being a linear or branched alkyl rest (C1 to C9), aryl rest (C1 to C9) or alkylaryl rest (C1 to C9), and
wherein X is selected from -(CF₂)₁₋₆-, -CF(CF₃)- and -CHF-CF₂-,
and wherein the surfactant is
selected from 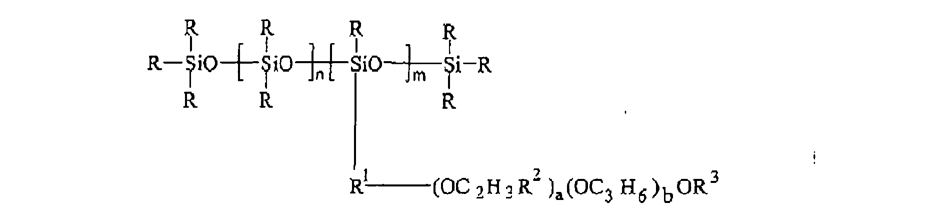 where each R is independently a monovalent hydrocarbyl radical with 1 to 22 C-atoms, R¹ is a divalent hydrocarbylene radical 1 to 26 C-atoms, each R² is independently hydrogen or a lower hydroxyalkyl radical, R³ is hydrogen or a monovalent hydrocarbyl radical with 1 to 22 C-atoms, n and b are independently greater than or equal to 0, and m and a are independently greater than or equal to 1, or
• Q-P-(OCₙH₂ₙ)ₓ-OT,
Q being R₃-Si- or R₃-Si-(R'-SiR₂)ₐ-R'-SiR"₂,
where each R in the molecule can be the same or different and stands for an aliphatic C₁-C₁₈, a cycloaliphatic C₆-C₁₂ or an aromatic C₆-C₁₂ hydrocarbon radical, which can optionally be substituted by halogen atoms; R' is a C₁-C₁₄ alkylene group; R" is R in the case of a≠0 or is R or R₃SiR' in the case of a=0, and a = 0 to 2; P stands for a C₂-C₁₈ alkylene group, or A-R"', where A represents a C₂-C₁₈ alkylene group and R'" a functional group from the following list: -NHC(O)-, -NHC(O) - (CH₂)ₙ₋₁-, -NHC(O)C(O)-, - NHC(O)(CH₂)ᵥC(O)-, -OC(O)-, -OC(O)-(CH₂)ₙ₋₁-, -OC(O)C(O)-, - OC(O)(CH₂)ᵥC(O)-, -OCH₂CH(OH)CH₂OC(O)(CH₂)ₙ₋₁-, - OCH₂CH(OH)CH₂OC(O)(CH₂)ᵥC(O)- with v = 1 to 12; T is H or stands for a C1 to C4 alkyl radical or a C1 to C4 acyll radical; x stands for a number from 1 to 200 and n stands for an average number from 1 to 6;
and mixtures thereof.

2. The dental composition according to claim 1, fulfilling at least one of the following parameters:
• Consistency (according to ISO 4823): 0, 1, 2 or 3,
• Setting time: within about 15 min after mixing at ambient conditions.

3. The dental composition according to any of the preceding claims, characterized after hardening by at least one of the following parameters:
• Tensile strength (according to DIN 53504): at least about 0.2 MPa,
• Elongation at break (according to DIN 53504): at least about 30 %,
• Recovery from deformation (according to DIN 53504): at least about 90%,
• Shore A hardness (according to DIN 53 505; 24h) at least about 20.

4. The dental composition of any of the preceding claims containing the F-containing compound in an amount effective to provide a composition having a water contact angle of less than about 20° at a water drop age of 10 s (Θ₁₀ₛ), 60 s after mixing of the components and/or having an initial water contact angle of less than about 80° (Θ₀₈), 40 s after mixing of the components.

5. The curable dental composition according to any of the preceding claims, wherein the F-containing compound is selected from the group of
• Rf-(O)ᵣ-CHF-(CF₂)ₓ-T, with t = 0 or 1, x = 0 or 1 and Rf being a linear br branched per- or partly fluorinated alkyl rest, wherein the alkyl chain can be interrupted by O atoms,
• Rf-(OCF₂)ₘ-O-CF₂-T, with m = 1 to about 6 and Rf being a linear or branched per- or partly fluorinated alkyl rest, wherein the alkyl chain can be interrupted by O atoms,
• CF₃-(CF₂)₂-(OCF(CF₃)-CF₂)_{z}O-L-T, with z = 0, 1, 2, 3, 4, 5, 6, 7 or 8 and L having a structure selected from -CF(CF₃)-, -CF_{z}- and -CF₂CF₂-,
• Rf-(O-CF₂CF₂)ₙ-O-CF₂-T, with n = 1, 2, 3, 4 or 5 and Rf being a linear or branched per- or partly fluorinated alkyl rest, wherein the alkyl chain can be interrupted by O atoms,
• an oligomeric compound obtainable by the polymerization or copolymerisation of monomers selected from the group of vinylidenfluoride, hexafluoropropylenoxide, tetrafluoroethylene, 2,2,3,3-tetrafluorooxetane, trifluoroethylene and monofluoroethylene, wherein at least one chaln-end of the oligomeric compound is represented by function T,
and mixtures thereof,
T being selected from the group consisting of -F, -COOR,-CH₂OR, -CHFOR,-CF₂OR, -CFHOH, -CH₂OH with R being a linear or branched alkyl rest (C1 to C9), aryl rest (C1 to C9) or alkylaryl rest (C1 to C9).

6. The dental composition of any of the preceding claims, wherein the F-containing compound is selected from
• Rf-O-CHF-T, with Rf being a linear or branched per- or partly fluorinated alkyl chain (C1 to C6), which can be interrupted by oxygen atoms,
• Rf-O-CF₂-T, with Rf being a linear or branched per- or partly fluorinated alkyl chain (C1 to C6), which can be interrupted by oxygen atoms,
• Rf-O-CHF-CF₂-T, with Rf being a linear or branched per- or partly fluorinated alkyl chain (C1 to C6), which can be interrupted by oxygen atoms,
• Rf-O-CF₂-CF₂-T, with Rf being a linear or branched per- or partly fluorinated alkyl chain (C1 to C6), which can be interrupted by oxygen atoms,
• R₁-O-CF₂-CHF-T, with Rf being a linear or branched per- or partly fluorinated alkyl chain (C1 to C6), which can be interrupted by oxygen atoms,
• Rf-O-CF₂-CHF-CF₂-T, with Rf being a linear or branched per- or partly fluorinated alkyl chain (C1 to C6), which can be interrupted by oxygen atoms,
• Rf-(O-CF₂)ᵤ-O-CF₂-T, with Rf being a linear or branched per- or partly fluorinated alkyl chain (C1 to C6), which can be interrupted by oxygen atoms, and u = 1, 2, 3, 4, 5 or 6,
• Rf-(O-CF₂-CF₂)ₖ-O-CF₂-T, with Rf being a linear or branched per- or partly fluorinated alkyl chain (C1 to C6), which can be interrupted by oxygen atoms, and k= 1, 2, 3, 4 or 5,
• Rf-(O-CF₂-CF₂-CF₂)ₙ-O-CF₂-CF₂-T, with n = 1 to 25 and Rf being a linear or branched per- or partly fluorinated alkyl chain (C1 to C6), wherein the alkyl chain can be interrupted by O atoms,
• T-CF₂-O-(CF₂-CF₂-O)ₚ-(CF₂-O)_{q}-CF₂-T, with p/q = 0.5 to 3,0 and an molecular weigth in the range of 500 to 4000 g/mol,
• T-CF₂-(O-CF(CF₃)-CF₂)ₙ-(O-CF₂)ₘ-O-CF₂-T, with n/m = 20-40 and a molecular weight in the range of 650 to 3200 g/mol,
• CF₃-(CF₂)₂-(OCF(CF₃)-CF₂)₂-O-CF(CF₃)-T, with z = 0, 9, 2, 3, 4, 5, 6, 7 or 8,
• CF₃-CHF-O-(CF₂)ₒ-T, with o = 1, 2, 3, 4, 5 or 6,
• CF₃-CF₂-O-(CF2)ₒ-T, with o = 9, 2, 3, 4, 5 or 6,
and mixtures thereof,
T being selected from the group consisting of -COOR, -CH₂OR, -CHFOR, - CF₂OH, -CFHOH, -CH₂OH or -F, and mixtures thereof, with R being a linear or branched alkyl rest (C1 to C9), aryl rest (C1 to C9) or alkylaryl rest (C1 to C9).

7. The dental composition of any of the preceding claims, wherein the F-containing compound is selected from
CF₃-O-CF₂-O-CF₂-CF₂-O-CHF-T
CF₃-(O-CF₂)₂-O-CF₂-CF₂-O-CHF-T
CF₃-(O-CF₂)₃-O-CF₂-CF₂-O-CHF-T
CF₃-O-CF₂-O-CF₂-CF₂-O-CHF-CF₂-T
CF₃-(O-CF₂)₂-O-CF₂-CF₂-O-CHF-CF₂-T
CF₃-(O-CF₂)₃-O-CF₂-CF₂-O-CHF-CF₂-T
C₃F₇-O-CF₂-CHF-T
CF₃-O-CF₂-O-CF₂-CF₂-O-CF₂-CHF-T
CF₃-(O-CF₂)₂-O-CF₂-CF₂-O-CF₂-CHF-T
CF₃-(O-CF₂)₃-O-CF₂-CF₂-O-CF₂-CHF-T
C₃F₇-O-CF₂-CHF-CF₂-T
CF₃-O-CF₂-CF₂-CF₂-O-CF₂-CHF-CF₂-T
CF₃-O-CF₂-O-CF₂-CF₂-O-CF₂-CHF-CF₂-T
CF₃-(C-CF₂)₂-O-CF₂-CF₂-O-CF₂-CHF-CF₂-T
CF₃-(O-CF₂)₃-O-CF₂-CF₂-O-CF₂-CHF-CF₂-T
CF₃-(O-CF₂)₃-O-CF₂-T
CF₃-(O-CF₂)₅-O-CF₂-T
C₂F₅-(O-CF₂-CF₂)₁-O-CF₂-T
C3F₇(O-CF₂-CF₂)₁-O-CF₂-T
C₄F₉-(O-CF₂-CF₂)₁-O-CF₂-T
C₂F₆-(O-CF₂-CF₂)2-O-CF₂-T
CF₃-(O-CF₂-CF₂)₂-O-CF₂-T
C₃F₇-(O-CF₂-CF₂)₂-O-CF₂-T
C₄F₃-(O-CF₂-CF₂)₂-O-CF₂-T
CF₃-(CF₂)₂-(O-CF(CF₃)-CF₂)₂-O-CF(CF₃)-T
CF₃-(CF₂)₂-(O-CF(CF₃)-CF₂)₃-O-CF(CF₃)-T
CF₃-(CF₂)₂-(O-CF(CF₃)-CF₂)₄-O-CF(CF₃)-T
CF₃-(CF₂)₂(O-CF(CF₃)-CF₂)₅-O-CF(CF₃)-T
CF₃-(CF₂)₂-(O-CF(CF₃)-CF₂)₆-O-CF(CF₃)-T
CF₃-CFH-O-(CF₂)₃-T
CF₃-CFH-O-(CF₂)₅-T
CF₃-CF₂-(CF₂)₃-T
CF₃-CF₂-O-(CF₂)₅-T
Rf-(O-CF₂-CF₂-CF₂)ₙ-O-CF₂-CF₂-T with n = 1 to 25 and Rf being a linear or branched per- or partly fluorinated alkyl rest (C1 to C6), wherein the alkyl chain can be interrupted by 0 atoms,
T-CF₂-O-(CF₂-CF₂-O)ₚ-(CF₂-O)_{q}-CF₂-T, with p/q = 0.5 to 3.0 and an molecular weight in the range of 500 to 4000 g/mol,
T-CF₂-(O-CF(CF₃)-CF₂)ₙ- (O-CF₂)ₘ-O-CF₂-T with n/m = 20 to 40 and a molecular weight in the range of 650 to 3200 g/mol,
and mixtures thereof,
T being selected from the group consisting of -COOR,-CH₂OR, -CHFOR, - CF₂OR, -CFHOH, -CH₂OH, and mixtures thereof, with R being a linear or branched alkyl rest (C1 to C9), aryl rest (C1 to C9) or alkylaryl rest (C1 to C9).

8. The dental composition of any of the preceding claims, wherein the molar ratio of F-containing compound to surfactant is in a range of 0.1 to 4.

9. The dental composition of any of the preceding claims comprising at least one of the following components:
f. filter as component (F),
g. at least one polydimethylsiloxane without aliphatically unsaturated groups as component (G),
h. additives as component (H) selected from retarders, rheology modifiers, inhibitors, pigments, plastizers, dyes, pigments, odorous substances, flavourings, stabilizers, hydrogen scavenger alone or in admixture.

10. The dental composition of claim 9, wherein the components are present in the following amounts:
• Component (A): from 20 wt.-% to 80 wt.-%,
• Component (B): from 0.1 wt.-% to 15 wt.-%,
• Component (C): from 0.001 wt.-% to 0.1 wt.-%,
• Component (D): from 0.1 wt.-% to 5 wt.-%,
• Component (E): from 0.1 wt.-% to 5 wt.-%,
• Component (F): from 0 wt.-% to 75 wt.-%,
• Component (G): from 0 wt.-% to 10 wt.-%,
• Component (H): from 0 wt.-% to 10 wt.-%,
wt.-% with respect to the whole composition.

11. A kit of parts comprising a base paste and a catalyst paste separated from each other before use, wherein the base paste comprises components (A) and (B) and the catalyst paste comprises component (C) or (C) and (A) and wherein component (D) and/or (E) and the other optional components (F), (G) and (H) can be present either in the base paste or the catalyst paste or the base paste and the catalyst paste, wherein components (A) to (H) are as described in any of the preceding claims.

12. The kit of parts according to claim 11, wherein component (D) is present in the base paste and component (E) is present in the catalyst paste.

13. A method of producing a dental composition comprising the step of combining the 13. A method of producing a dental composition comprising the step of combining the F-containing compound with a hardenable matrix comprising a surfactant, wherein the surfactant and the F-containing compound are as described in any of claims 1 to 8.

14. Use of the dental composition as described in any of claims 1 to 10 or the kit according to any of claims 11 or 12 for the preparation of or as impression material or for the preparation of crowns and bridges.

15. Use of the F-containing component as described in any of claims 1 to 7 for enhancing the wettability of a hardenable composition as described in any of claims 1 to 10.

## Patentansprüche

1. Härtbare Dentalmasse, umfassend
a. ein härtbares Silikonpolymer als Komponente (A), wobei das Silikonpolymer mindestens zwei funktionelle Gruppen, die zur Reaktion mit einer SiH-Gruppe in Gegenwart eines Hydrosilylierungskatalysators befähigt sind, enthält,
b. eine Vemetzerverbindung, die mindestens zwei SiH-Gruppen enthält, als Komponente (B),
c. einen Katalysator, der zur Katalyse einer Hydrosilylierungsreaktion befähigt ist, als Komponente (C),
d. ein Tensid als Komponente (D),
e. eine F-haltige Verbindung als Komponente (E), wobei die F-haltige Verbindung die folgende Formel aufweist:
T₁-X-[(O-CF₂-CF₂)ᵤ-(O-CF₂)ᵥ-(O-CF(CF₃)-CF₂)_{w}-(O-CF₂-CF₂-CF₂)ₓ-O]-X-T₂
mit u = 0 bis 8, v = 0 bis 8, w = 0 bis 8 und x = 0 bis 8 und u + v + w + x ≥ 1,
wobei T₁ und T₂ gleich oder verschieden sein können und unabhängig voneinander unter -COOR, -CH₂OH, -CF₂OR, -CHFOH, -CHFOR, -CH₂OR oder -F ausgewählt sind, wobei R für einen linearen oder verzweigten Alkylrest (C1 bis C9), Arylrest (C1 bis C9) oder Alkylarylrest (C1 bis C9) steht, und
wobei X unter -(CF₂)₁₋₈-, -CF(CF₃)- und -CHF-CF₂- ausgewählt ist, und
wobei das Tensid unter
• 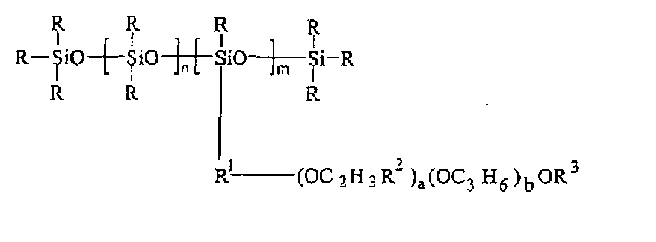 wobei R jeweils unabhängig für einen einwertigen Hydrocarbylrest mit 1 bis 22 C-Atomen steht, R¹ für einen zweiwertigen Hydrocarbylenrest mit 1 bis 26 C-Atomen steht, R² jeweils unabhängig für Wasserstoff oder einen Niederhydroxyalkylrest steht, R³ für Wasserstoff oder einen einwertigen Hydrocarbylrest mit 1 bis 22 C-Atomen steht, n und b unabhängig voneinander größer gleich 0 sind und m und a unabhängig voneinander größer gleich 1 sind, oder
• Q-P-(OCₙH₂ₙ)ₓ-OT,
wobei Q für R₃-Si- oder R₃-Si-(R'-SiR₂)ₐ-R'-SiR"₂ steht,
wobei jedes R in dem Molekül gleich oder verschieden sein kann und für einen aliphatischen C₁-C₁₈-, einen cycloaliphatischen C₆-C₁₂- oder einen aromatischen C₆-C₁₂-Kohlenwasserstoffrest, der gegebenenfalls durch Halogenatome substituiert sein kann, steht; R' für eine C₁-C₁₄-Alkylengruppe steht; R" im Fall von a ≠ 0 für R steht oder im Fall von a = 0 für R₃SiR' steht, und a = 0 bis 2; P für eine C₂-C₁₈-Alkylengruppe oder A-R'" steht, wobei A für eine C₂-C₁₈-Alkylengruppe steht und R'" für eine funktionelle Gruppe aus der folgenden Liste steht: -NHC(O)-, -NHC(O)-(CH₂)ₙ₋₁-, -NHC(O)C(O)-, -NHC(O)(CH₂)ᵥC(O)-, -OC(O)-, -OC(O)-(CH₂)ₙ₋₁-, -OC(O)C(O)-, -OC(O)(CH₂)ᵥC(O)-, -OCH₂CH(OH)CH₂OC(O)(CH₂)ₙ₋₁-, -OCH₂CH(OH)CH₂OC(O)(CH₂)ᵥC(O)- mit v = 1 bis 12; T für H oder einen C1- bis C4-Alkylrest oder einen C1- bis C4-Acylrest steht; x für eine Zahl von 1 bis 200 steht und n für eine durchschnittliche Zahl von 1 bis 6 steht;
und Mischungen davon ausgewählt ist.

2. Dentalmasse nach Anspruch 1, die mindestens einen der folgenden Parameter erfüllt:
• Konsistenz (gemäß ISO 4823): 0, 1, 2 oder 3,
• Abbindezeit: innerhalb von etwa 15 Minuten nach Mischen bei Umgebungsbedingungen.

3. Dentalmasse nach einem der vorhergehenden Ansprüche, die nach Härtung durch mindestens einen der folgenden Parameter gekennzeichnet ist:
• Zugfestigkeit (gemäß DIN 53504): mindestens etwa 0,2 MPa,
• Reißdehnung (gemäß DIN 53504): mindestens etwa 30%,
• Rückstellung nach Verformung (gemäß DIN 53504): mindestens etwa 90%,
• Shore-A-Härte (gemäß DIN 53505; 24 h): mindestens etwa 20.

4. Dentalmasse nach einem der vorhergehenden Ansprüche, die die F-haltige Verbindung in einer zur Bereitstellung einer Zusammensetzung mit einem Wasserkontaktwinkel von weniger als etwa 20° bei einem Wassertropfenalter von 10 s (Θ₁₀ₛ) 60 s nach dem Mischen der Komponenten und/oder mit einem initialen Wasserkontaktwinkel von weniger als etwa 80° (Θ₀ₛ) 40 s nach dem Mischen der Komponenten wirksamen Menge enthält.

5. Härtbare Dentalmasse nach einem der vorhergehenden Ansprüche, wobei die F-haltige Verbindung aus der Gruppe
• Rf-(O)ₜ-CHF-(CF₂)ₓ-T mit t = 0 oder 1 und x = 0 oder 1 und wobei Rf ein linearer oder verzweigter per- oder teilfluorierter Alkylrest ist, wobei die Alkylkette durch O-Atome unterbrochen sein kann,
• Rf-(OCF₂)ₘ-O-CF₂-T mit m = 1 bis etwa 6 und wobei Rf ein linearer oder verzweigter per- oder teilfluorierter Alkylrest ist, wobei die Alkylkette durch O-Atome unterbrochen sein kann,
• CF₃-(CF₂)₂-(OCF(CF₃)-CF₂)_{z}-O-L-T mit z = 0, 1, 2, 3, 4, 5, 6, 7 oder 8 und wobei L eine unter -CF(CF₃)-, -CF₂- und -CF₂CF₂- ausgewählte Struktur aufweist,
• Rf-(O-CF₂CF₂)ₙ-O-CF₂-T mit n = 1, 2, 3, 4 oder 5 und wobei Rf ein linearer oder verzweigter per- oder teilfluorierter Alkylrest ist, wobei die Alkylkette durch O-Atome unterbrochen sein kann,
• einer oligomeren Verbindung, die durch Polymerisation oder Copolymerisation von Monomeren aus der Gruppe Vinylidenfluorid, Hexafluorpropylenoxid, Tetrafluorethylen, 2,2,3,3-Tetrafluoroxetan, Trifluorethylen und Monofluorethylen erhältlich ist, wobei mindestens ein Kettenende der oligomeren Verbindung durch die Funktion T wiedergegeben wird,
und Mischungen davon ausgewählt ist,
wobei T aus der Gruppe bestehend aus -F, -COOR, -CH₂OR, -CHFOR, -CF₂OR, -CFHOH, -CH₂OH ausgewählt ist, wobei R für einen linearen oder verzweigten Alkylrest (C1 bis C9), Arylrest (C1 bis C9) oder Alkylarylrest (C1 bis C9) steht.

6. Dentalmasse nach einem der vorhergehenden Ansprüche, wobei die F-haltige Verbindung unter
• Rf-O-CHF-T, wobei Rf eine lineare oder verzweigte per- oder teilfluorierte Alkylkette (C1 bis C6) ist, die durch Sauerstoffatome unterbrochen sein kann,
• Rf-O-CF₂-T, wobei Rf eine lineare oder verzweigte per- oder teilfluorierte Alkylkette (C1 bis C6) ist, die durch Sauerstoffatome unterbrochen sein kann,
• Rf-O-CHF-CF₂-T, wobei Rf eine lineare oder verzweigte per- oder teilfluorierte Alkylkette (C1 bis C6) ist, die durch Sauerstoffatome unterbrochen sein kann,
• Rf-O-CF₂-CF₂-T, wobei Rf eine lineare oder verzweigte per- oder teilfluorierte Alkylkette (C1 bis C6) ist, die durch Sauerstoffatome unterbrochen sein kann,
• Rf-O-CF₂-CHF-T, wobei Rf eine lineare oder verzweigte per- oder teilfluorierte Alkylkette (C1 bis C6) ist, die durch Sauerstoffatome unterbrochen sein kann,
• Rf-O-CF₂-CHF-CF₂-T, wobei Rf eine lineare oder verzweigte per- oder teilfluorierte Alkylkette (C1 bis C6) ist, die durch Sauerstoffatome unterbrochen sein kann,
• Rf-(O-CF₂)ᵤ-O-CF₂-T, wobei Rf eine lineare oder verzweigte per- oder teilfluorierte Alkylkette (C1 bis C6) ist, die durch Sauerstoffatome unterbrochen sein kann, und u = 1, 2, 3, 4, 5 oder 6,
• Rf-(O-CF₂-CF₂)ₖ-O-CF₂-T, wobei Rf eine lineare oder verzweigte per- oder teilfluorierte Alkylkette (C1 bis C6) ist, die durch Sauerstoffatome unterbrochen sein kann, und k = 1, 2, 3, 4 oder 5,
• Rf-(O-CF₂-CF₂-CF₂)ₙ-O-CF₂-CF₂-T mit n = 1 bis 25 und wobei Rf eine lineare oder verzweigte per- oder teilfluorierte Alkylkette (C1 bis C6) ist, wobei die Alkylkette durch O-Atome unterbrochen sein kann,
• T-CF₂-O-(CF₂-CF₂-O)p-(CF₂-O)_{q}-CF₂-T mit p/q = 0,5 bis 3,0 und einem Molekulargewicht im Bereich von 500 bis 4000 g/mol,
• T-CF₂-(O-CF(CF₃)-CF₂)ₙ-(O-CF₂)ₘ-O-CF₂-T mit n/m = 20-40 und einem Molekulargewicht im Bereich von 650 bis 3200 g/mol,
• CF₃-(CF₂)₂-(OCF(CF₃)-CF₂)₂-O-CF(CF₃)-T mit z = 0, 1, 2, 3, 4, 5, 6, 7 oder 8,
• CF₃-CHF-O-(CF₂)ₒ-T mit o = 1, 2, 3, 4, 5 oder 6,
• CF₃-CF₂-O-(CF₂)ₒ-T mit o = 1, 2, 3, 4, 5 oder 6 und Mischungen davon ausgewählt ist,
wobei T aus der Gruppe bestehend aus -COOR, -CH₂OR, -CHFOR,
-CF₂OH, -CFHOH, -CH₂OH oder -F und Mischungen davon ausgewählt ist, wobei R für einen linearen oder verzweigten Alkylrest (C1 bis C9), Arylrest (C1 bis C9) oder Alkylarylrest (C1 bis C9) steht.

7. Dentalmasse nach einem der vorhergehenden Ansprüche, bei dem die F-haltige Verbindung unter
CF₃-O-CF₂-O-CF₂-CF₂-O-CHF-T
CF₃-(O-CF₂)₂-O-CF₂-CF₂-O-CHF-T
CF₃-(O-CF₂)₃-O-CF₂-CF₂-O-CHF-T
CF₃-O-CF₂-O-CF₂-CF₂-O-CHF-CF₂-T
CF₃-(O-CF₂)₂-O-CF₂-CF₂-O-CHF-CF₂-T
CF₃-(O-CF₂)₃-O-CF₂-CF₂-O-CHF-CF₂-T
C₃F₇-O-CF₂-CHF-T
CF₃-O-CF₂-O-CF₂-CF₂-O-CF₂-CHF-T
CF₃-(O-CF₂)₂-O-CF₂-CF₂-O-CF₂-CHF-T
CF₃-(O-CF₂)₃-O-CF₂-CF₂-O-CF₂-CHF-T
C₃F₇-O-CF₂-CHF-CF₂-T
CF₃-O-CF₂-CF₂-CF₂-O-CF₂-CHF-CF₂-T
CF₃-O-CF₂-O-CF₂-CF₂-O-CF₂-CHF-CF₂-T
CF₃-(O-CF₂)₂-O-CF₂-CF₂-O-CF₂-CHF-CF₂-T
CF₃-(O-CF₂)₃-O-CF₂-CF₂-O-CF₂-CHF-CF₂-T
CF₃-(O-CF₂)₃-O-CF₂-T
CF₃-(O-CF₂)₅-O-CF₂-T
C₂F₅-(O-CF₂-CF₂)₁-O-CF₂-T
C₃F₇-(O-CF₂-CF₂)₁-O-CF₂-T
C₄F₉-(O-CF₂-CF₂)₁-O-CF₂-T
C₂F₅-(O-CF₂-CF₂)₂-O-CF₂-T
CF₃-(O-CF₂-CF₂)₂-O-CF₂-T
C₃F₇-(O-CF₂-CF₂)₂-O-CF₂-T
C₄F₉-(O-CF₂-CF₂)₂-O-CF₂-T
CF₃-(CF₂)₂-(O-CF(CF₃)-CF₂)₂-O-CF(CF₃)-T
CF₃-(CF₂)₂-(O-CF(CF₃)-CF₂)₃-O-CF(CF₃)-T
CF₃-(CF₂)₂-(O-CF(CF₃)-CF₂)₄-O-CF(CF₃)-T
CF₃-(CF₂)₂-(O-CF(CF₃)-CF₂)₅-O-CF(CF₃)-T
CF₃-(CF₂)₂-(O-CF(CF₃)-CF₂)₆-O-CF(CF₃)-T
CF₃-CFH-O-(CF₂)₃-T
CF₃-CFH-O-(CF₂)₅-T
CF₃-CF₂-O-(CF₂)₃-T
CF₃-CF₂-O-(CF₂)₅-T
Rf-(O-CF₂-CF₂-CF₂)ₙ-O-CF₂-CF₂-T mit n = 1 bis 25 und wobei Rf ein linearer oder verzweigter per- oder teilfluorierter Alkylrest (C1 bis C6) ist, wobei die Alkylkette durch O-Atome unterbrochen sein kann, T-CF₂-O-(CF₂-CF₂-O)ₚ-(CF₂-O)_{q}-CF₂-T mit p/q = 0,5 bis 3,0 und einem Molekulargewicht im Bereich von 500 bis 4000 g/mol, T-CF₂-(O-CF(CF₃)-CF₂)ₙ-(O-CF₂)ₘ-O-CF₂-T mit n/m = 20 bis 40 und einem Molekulargewicht im Bereich von 650 bis 3200 g/mol,
und Mischungen davon ausgewählt ist,
wobei T aus der Gruppe bestehend aus -COOR, -CH₂OR, -CHFOR, -CF₂OR, -CFHOH, -CH₂OH und Mischungen davon ausgewählt ist, wobei R für einen linearen oder verzweigten Alkylrest (C1 bis C9), Arylrest (C1 bis C9) oder Alkylarylrest (C1 bis C9) steht.

8. Dentalmasse nach einem der vorhergehenden Ansprüche, wobei das Molverhältnis von F-haltiger Verbindung zu Tensid im Bereich von 0,1 bis 4 liegt.

9. Dentalmasse nach einem der vorhergehenden Ansprüche, umfassend mindestens eine der folgenden Komponenten:
f. Füllstoff als Komponente (F),
g. mindestens ein Polydimethylsiloxan ohne aliphatisch ungesättigte Gruppen als Komponente (G),
h. Additive als Komponente (H), ausgewählt unter Verzögerern, Rheologiemodifikatoren, Inhibitoren, Pigmenten, Weichmachern, Farbstoffen, Geruchsstoffen, Geschmacksstoffen, Stabilisatoren und Wasserstoff-Fängern alleine oder im Gemisch.

10. Dentalmasse nach Anspruch 9, wobei die Komponenten in den folgenden Mengen vorliegen:
• Komponente (A): von 20 Gew.-% bis 80 Gew.-%,
• Komponente (B): von 0,1 Gew.-% bis 15 Gew.-%,
• Komponente (C): von 0,001 Gew.-% bis 0,1 Gew.-%,
• Komponente (D): von 0,1 Gew.-% bis 5 Gew.-%,
• Komponente (E): von 0,1 Gew.-% bis 5 Gew.-%,
• Komponente (F): von 0 Gew.-% bis 75 Gew.-%,
• Komponente (G): von 0 Gew.-% bis 10 Gew.-%,
• Komponente (H): von 0 Ges.-% bis 10 Ges.-%,
wobei sich Gew.-% auf die gesamte Zusammensetzung bezieht.

11. Kit von Teilen, umfassend eine Basispaste und eine Katalysatorpaste, die vor der Verwendung voneinander getrennt sind, wobei die Basispaste die Komponenten (A) und (B) umfasst und die Katalysatorpaste die Komponente (C) oder (C) und (A) umfasst und wobei die Komponente (D) und/oder (E) und die anderen fakultativen Komponenten (F), (G) und (H) entweder in der Basispaste oder in der Katalysatorpaste oder der Basispaste und der Katalysatorpaste vorliegen können, wobei die Komponenten (A) bis (H) der Beschreibung in einem der vorhergehenden Ansprüche entsprechen.

12. Kit von Teilen nach Anspruch 11, wobei die Komponente (D) in der Basispaste vorliegt und die Komponente (E) in der Katalysatorpaste vorliegt.

13. Verfahren zur Herstellung einer Dentalmasse, bei dem man die F-haltige Verbindung mit einer härtbaren Matrix, die ein Tensid umfasst, kombiniert, wobei das Tensid und die F-haltige Verbindung der Beschreibung in einem der Ansprüche 1 bis 8 entsprechen.

14. Verwendung der Dentalmasse gemäß einem der Ansprüche 1 bis 10 oder des Kits gemäß einem der Ansprüche 11 oder 12 zur Herstellung von oder als Abformmaterial oder zur Herstellung von Kronen und Brücken.

15. Verwendung der F-haltigen Komponente gemäß einem der Ansprüche 1 bis 7 zur Verbesserung des Benetzungsvermögens einer härtbaren Zusammensetzung gemäß einem der Anprüche 1 bis 10.

## Revendications

1. Composition dentaire durcissable comprenant :
a. un polymère de silicone durcissable au titre de composant (A), le polymère de silicone contenant au moins deux groupements fonctionnels pouvant réagir avec un groupement SiH en présence d'un catalyseur d'hydrosilylation,
b. un agent de réticulation contenant au moins deux groupements SiH au titre de composant (B),
c. un catalyseur pouvant catalyser une réaction d'hydrosilylation au titre de composant (C),
d. un tensioactif au titre de composant (D),
e. un composé fluoré au titre de composant (E), le composé fluoré répondant à la formule suivante :
T₁-X-[(O-CF₂-CF₂)ᵤ-(O-CF₂)ᵥ-(O-CF(CF₃)-CF₂)_{w}-(O-CF₂-CF₂-CF₂)ₓ-O]-X-T₂
avec u = 0 à 8, v = 0 à 8, w = 0 à 8 et x = 0 à 8 et u + v + w + x > 1,
où chacun des radicaux T₁ et T₂ peut être identique ou différent et est indépendamment choisi parmi les groupements -COOR, -CH₂OH, -CF₂OR, -CHFOH, -CHFOR, -CH₂OR ou -F, R représentant un résidu alkyle linéaire ou ramifié en C1 à C9, un résidu aryle en C1 à C9 ou un résidu alkylaryle en C1 à C9,
et
où X est choisi parmi -(CF₂)₁₋₈-, -CF(CF₃)- et -CHF-CF₂-,
et où le tensioactif est choisi parmi
• 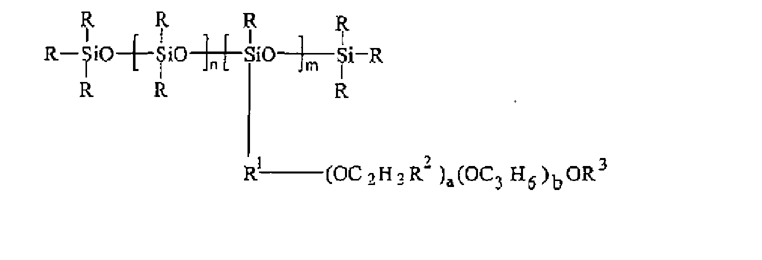 où chacun des radicaux R représente indépendamment un radical hydrocarbyle monovalent comportant entre 1 et 22 atomes C, R¹ représente un radical hydrocarbylène divalent comportant entre 1 et 26 atomes C, chacun des radicaux R² représente indépendamment un atome d'hydrogène ou un radical hydroxyalkyle court, R³ représente un atome d'hydrogène ou un radical hydrocarbyle monovalent comportant entre 1 et 22 atomes C, chacun des nombres n et b est indépendamment supérieur ou égal à 0, et chacun des nombres m et a est indépendamment supérieur ou égal à 1, ou
• Q-P-(OCₙH₂ₙ)ₓ-OT,
Q représentant R₃-Si- ou R₃-Si-(R'-SiR₂)ₐ-R'-SiR"₂,
chacun des radicaux R de la molécule pouvant être identique ou différent et
représentant un radical hydrocarboné aliphatique en C₁-C₁₈, cycloaliphatique en C₆-C₁₂ ou aromatique en C₆-C₁₂, qui peut être éventuellement substitué par des atomes d'halogène ; R' représente un groupement alkylène en C₁-C₁₄ ; R" représente R dans le cas où a ≠ 0 ou représente R ou R₃SiR' dans le cas où a = 0, et
a = 0 à 2 ; P représente un groupement alkylène en C₂-C₁₈ ou A-R"', où A représente un groupement alkylène en C₂-C₁₈ et R"' représente un groupement fonctionnel de la liste suivante : -NHC(O)-, -NHC(O) - (CH₂)ₙ₋₁-, -NHC(O)C(O)-, -NHC(O)(CH₂)ᵥC(O)-, -OC(O)-, -OC(O)-(CH₂)ₙ₋₁-, -OC(O)C(O)-, -OC(O)(CH₂)ᵥC(O)-, -OCH₂CH(OH)CH₂OC(O)(CH₂)ₙ₋₁-,
-OCH₂CH(OH)CH₂OC(O)(CH₂)ᵥC(O)- avec v = 1 à 12 ; T représente H, un radical alkyle en C1 à C4 ou un radical acyle en C1 à C4 ; x représente un nombre compris entre 1 et 200 et n représente un nombre moyen compris entre 1 et 6 ;
et leurs mélanges.

2. Composition dentaire conforme à la revendication 1, répondant à au moins un des paramètres suivants :
• Consistance (selon ISO 4823) : 0, 1, 2 ou 3,
• Durée de prise : dans les 15 minutes environ après mélangeage dans des conditions ambiantes.

3. Composition dentaire conforme à l'une quelconque des revendications précédentes, caractérisée après la prise par au moins l'un des paramètres suivants :
• Résistance à la traction (selon DIN 53504) : au moins environ 0,2 MPa,
• Allongement à la rupture (selon DIN 53504) : au moins environ 30 %,
• Récupération après déformation (selon DIN 53504) : au moins environ 90 %,
• Dureté Shore A (selon DIN 53505 ; 24 h) : au moins environ 20.

4. Composition dentaire conforme à l'une quelconque des revendications précédentes qui contient le composé fluoré en une teneur suffisante pour obtenir une composition d'angle de contact avec l'eau inférieur à environ 20° à une durée de vie de goutte d'eau de 10 secondes (θ₁₀ₛ), 60 secondes après mélangeage des composants et/ou d'angle de contact initial avec l'eau inférieur à environ 80° (θ₀ₛ), 40 secondes après mélangeage des composants.

5. Composition dentaire durcissable conforme à l'une quelconque des revendications précédentes, où le composé fluoré est choisi dans le groupe constitué par :
• Rf-(O)ₜ-CHF-(CF₂)ₓ-T, avec t = 0 ou 1, x = 0 ou 1 et Rf un résidu alkyle linéaire ou ramifié perfluoré ou partiellement fluoré, la chaîne alkylique pouvant être interrompue par des atomes O,
• Rf-(OCF₂)ₘ-O-CF₂-T, avec m = 1 à environ 6 et Rf un résidu alkyle linéaire ou ramifié perfluoré ou partiellement fluoré, la chaîne alkylique pouvant être interrompue par des atomes O,
• CF₃-(CF₂)₂-(OCF(CF₃)-CF₂)_{z}-O-L-T, avec z = 0, 1, 2, 3, 4, 5, 6, 7 ou 8 et L représentant une structure choisie parmi -CF(CF₃)-, -CF₂- et -CF₂CF₂-,
• Rf-(O-CF₂CF₂)ₙ-O-CF₂-T, avec n = 1, 2, 3, 4 ou 5 et Rf un résidu alkyle linéaire ou ramifié perfluoré ou partiellement fluoré, la chaîne alkylique pouvant être interrompue par des atomes O,
• un composé oligomère pouvant être obtenu par la polymérisation ou la copolymérisation de monomères choisis dans le groupe du fluorure de vinylidène, de l'hexafluoropropylène oxyde, du tétrafluoroéthylène, du 2,2,3,3-tétrafluoroxétanne, du trifluoroéthylène et du monofluoroéthylène, où au moins une extrémité de chaîne du composé oligomère est représentée par la fonction T,
et leurs mélanges,
T étant choisi dans le groupe constitué par les groupements -F, -COOR, -CH₂OR, -CHFOR, -CF₂OR, -CFHOH, -CH₂OH, R représentant un résidu alkyle linéaire ou ramifié en C1 à C9, un résidu aryle en C1 à C9 ou un résidu alkylaryle en C1 à C9.

6. Composition dentaire conforme à l'une quelconque des revendications précédentes, où le composé fluoré est choisi parmi les suivants :
• Rf-O-CHF-T, avec Rf une chaîne alkylique linéaire ou ramifiée perfluorée ou partiellement fluorée en C1 à C6, qui peut être interrompue par des atomes d'oxygène,
• Rf-O-CF₂-T, avec Rf une chaîne alkylique linéaire ou ramifiée perfluorée ou partiellement fluorée en C1 à C6, qui peut être interrompue par des atomes d'oxygène,
• Rf-O-CHF-CF₂-T, avec Rf une chaîne alkylique linéaire ou ramifiée perfluorée ou partiellement fluorée en C1 à C6, qui peut être interrompue par des atomes d'oxygène,
• Rf-O-CF₂-CF₂-T, avec Rf une chaîne alkylique linéaire ou ramifiée perfluorée ou partiellement fluorée en C1 à C6, qui peut être interrompue par des atomes d'oxygène,
• Rf-O-CF₂-CHF-T, avec Rf une chaîne alkylique linéaire ou ramifiée perfluorée ou partiellement fluorée en C1 à C6, qui peut être interrompue par des atomes d'oxygène,
• Rf-O-CF₂-CHF-CF₂-T, avec Rf une chaîne alkylique linéaire ou ramifiée perfluorée ou partiellement fluorée en C1 à C6, qui peut être interrompue par des atomes d'oxygène,
• Rf-(O-CF₂)ᵤ-O-CF₂-T, avec Rf une chaîne alkylique linéaire ou ramifiée perfluorée ou partiellement fluorée en C1 à C6, qui peut être interrompue par des atomes d'oxygène, et u = 1, 2, 3, 4, 5 ou 6,
• Rf-(O-CF₂-CF₂)ₖ-O-CF₂-T, avec Rf une chaîne alkylique linéaire ou ramifiée perfluorée ou partiellement fluorée en C1 à C6, qui peut être interrompue par des atomes d'oxygène, et k = 1, 2, 3, 4 ou 5,
• Rf-(O-CF₂-CF₂-CF₂)ₙ-O-CF₂-CF₂-T, avec n = 1 à 25 et Rf une chaîne alkylique linéaire ou ramifiée perfluorée ou partiellement fluorée en C1 à C6, la chaîne alkylique pouvant être interrompue par des atomes O,
• T-CF₂-O-(CF₂-CF₂-O)ₚ-(CF₂-O)_{q}-CF₂-T, avec p/q = 0,5 à 3,0 et de masse moléculaire comprise entre 500 et 4000 g/mol,
• T-CF₂-(O-CF(CF₃)-(CF₂)ₙ-(O-CF₂)ₘ-O-CF₂-T, avec n/m = 20 à 40 et de masse moléculaire comprise entre 650 et 3200 g/mol,
• CF₃-(CF₂)₂-(OCF(CF₃)-CF₂)_{z}-O-CF(CF₃)-T, avec z = 0, 1, 2, 3, 4, 5, 6, 7 ou 8,
• CF₃-CHF-O-(CF₂)ₒ-T, avec o = 1, 2, 3, 4, 5 ou 6,
• CF₃-CF₂-O-(CF₂)ₒ-T, avec o = 1, 2, 3, 4, 5 ou 6,
et leurs mélanges,
T étant choisi dans le groupe constitué par les groupements -COOR, -CH₂OR, -CHFOR, -CF₂OH, -CFHOH, -CH₂OH ou -F, et leurs mélanges R représentant un résidu alkyle linéaire ou ramifié en C1 à C9, un résidu aryle en C1 à C9 ou un résidu alkylaryle en C1 à C9.

7. Composition dentaire conforme à l'une quelconque des revendications précédentes, où le composé fluoré est choisi parmi les suivants :
CF₃-O-CF₂-O-CF₂-CF₂-O-CHF-T
CF₃-(O-CF₂)₂-O-CF₂-CF₂-O-CHF-T
CF₃-(O-CF₂)₃-O-CF₂-CF₂-O-CHF-T
CF₃-O-CF₂-O-CF₂-CF₂-O-CHF-CF₂-T
CF₃-(O-CF₂)₂-O-CF₂-CF₂-O-CHF-CF₂-T
CF₃-(O-CF₂)₃-O-CF₂-CF₂-O-CHF-CF₂-T
C₃F₇-O-CF₂-CHF-T
CF₃-O-CF₂-O-CF₂-CF₂-O-CF₂-CHF-T
CF₃-(O-CF₂)₂-O-CF₂-CF₂-O-CF₂-CHF-T
CF₃-(O-CF₂)₃-O-CF₂-CF₂-O-CF₂-CHF-T
C₃F₇-O-CF₂-CHF-CF₂-T
CF₃-O-CF₂-CF₂-CF₂-O-CF₂-CHF-CF₂-T
CF₃-O-CF₂-O-CF₂-CF₂-O-CF₂-CHF-CF₂-T
CF₃-(O-CF₂)₂-O-CF₂-CF₂-O-CF₂-CHF-CF₂-T
CF₃-(O-CF₂)₃-O-CF₂-CF₂-O-CF₂-CHF-CF₂-T
CF₃-(O-CF₂)₃-O-CF₂-T
CF₃-(O-CF₂)₅-O-CF₂-T
C₂F₅-(O-CF₂-CF₂)₁-O-CF₂-T
C₃F₇-(O-CF₂-CF₂)₁-O-CF₂-T
C₄F₉-(O-CF₂-CF₂)₁-O-CF₂-T
C₂F₅-(O-CF₂-CF₂)₂-O-CF₂-T
CF₃-(O-CF₂-CF₂)₂-O-CF₂-T
C₃F₇-(O-CF₂-CF₂)₂-O-CF₂-T
C₄F₉-(O-CF₂-CF₂)₂-O-CF₂-T
CF₃-(CF₂)₂-(O-CF(CF₃)-CF₂)₂-O-CF(CF₃)-T
CF₃-(CF₂)₂-(O-CF(CF₃)-CF₂)₃-O-CF(CF₃)-T
CF₃-(CF₂)₂-(O-CF(CF₃)-CF₂)₄-O-CF(CF₃)-T
CF₃-(CF₂)₂-(O-CF(CF₃)-CF₂)₅-0-CF(CF₃)-T
CF₃-(CF₂)₂-(O-CF(CF₃)-CF₂)₆-O-CF(CF₃)-T
CF₃-CFH-O-(CF₂)₃-T
CF₃-CFH-O-(CF₂)₅-T
CF₃-CF₂-O-(CF₂)₃-T
CF₃-CF₂-O-(CF₂)₅-T
Rf-(O-CF₂-CF₂-CF₂),-O-CF₂-CF₂-T, avec n = 1 à 25 et Rf un résidu alkyle linéaire ou ramifié perfluoré ou partiellement fluoré en C1 à C6, la chaîne alkylique pouvant être interrompue par des atomes O,
T-CF₂-O-(CF₂-CF₂-O)ₚ-(CF₂-O)_{q}-CF₂-T, avec p/q = 0,5 à 3,0 et de masse moléculaire comprise entre 500 et 4000 g/mol,
T-CF₂-(O-CF(CF₃)-CF₂)ₙ-(O-CF₂)ₘ-O-CF₂-T, avec n/m = 20 à 40 et de masse moléculaire comprise entre 650 et 3200 g/mol,
et leurs mélanges,
T étant choisi dans le groupe constitué par les groupements -COOR, -CH₂OR, - CHFOR, - CF₂OR, -CFHOH, -CH₂OH et leurs mélanges, R représentant un résidu alkyle linéaire ou ramifié en C1 à C9, un résidu aryle en C1 à C9 ou un résidu alkylaryle en C1 à C9.

8. Composition dentaire conforme à l'une quelconque des revendications précédentes, où le rapport molaire du composé fluoré sur le tensioactif est compris entre 0,1 et 4.

9. Composition dentaire conforme à l'une quelconque des revendications précédentes, comprenant au moins l'un des composés suivants :
f. une charge au titre de composant (F),
g. au moins un polydiméthylsiloxane sans groupements aliphatiquement insaturés au titre de composant (G),
h. des adjuvants au titre de composant (H) choisis parmi les suivants : agents retardateurs, agents rhéologiques, inhibiteurs, pigments, plastifiants, colorants, pigments, substances odorantes, arômes, agents stabilisants, capteurs d'hydrogène, seuls ou en mélange.

10. Composition dentaire conforme à la revendication 9, où les composants sont présents dans les quantités suivantes :
• Composant (A) : entre 20 % en masse et 80 % en masse,
• Composant (B) : entre 0,1 % en masse et 15 % en masse,
• Composant (C) : entre 0,001 % en masse et 0,1 % en masse,
• Composant (D) : entre 0,1 % en masse et 5 % en masse,
• Composant (E) : entre 0,1 % en masse et 5 % en masse,
• Composant (F) : entre 0 % en masse et 75 % en masse,
• Composant (G) : entre 0 % en masse et 10 % en masse,
• Composant (H) : entre 0 % en masse et 10 % en masse,
les pourcentages massiques étant donnés par rapport à la composition totale.

11. Kit composé de plusieurs parties et comprenant une pâte de base et une pâte catalytique séparées l'une de l'autre avant utilisation, où la pâte de base comprend les composants (A) et (B) et la pâte catalytique comprend les composants (C) ou (C) et (A), et où le composant (D) et/ou (E) et les autres composants optionnels (F), (G) et (H) peuvent être présents soit dans la pâte de base, soit dans la pâte catalytique ou dans la pâte de base et dans la pâte catalytique, les composants (A) à (H) étant tels que décrits dans l'une quelconque des revendications précédentes.

12. Kit composé de plusieurs parties conforme à la revendication 11, où le composant (D) est présent dans la pâte de base et le composant (E) est présent dans la pâte catalytique.

13. Procédé de production d'une composition dentaire comprenant l'étape de combinaison du composé fluoré avec une matrice durcissable comprenant un tensioactif, où le tensioactif et le composé fluoré sont tels que décrits dans l'une quelconque des revendications 1 à 8.

14. Utilisation de la composition dentaire conforme à l'une quelconque des revendications 1 à 10 ou du kit conforme à l'une quelconque des revendications 11 ou 12 dans la préparation d'un matériau d'impression, en tant que matériau d'impression, ou dans la préparation de couronnes et de bridges.

15. Utilisation du composant fluoré conforme à l'une quelconque des revendications 1 à 7 dans l'amélioration de la mouillabilité d'une composition durcissable conforme à l'une quelconque des revendications 1 à 10.
